# FASCICULE DE BREVET EUROPEEN

(11) **EP 1 294 400 B1**
(45) Date de publication et mention de la délivrance du brevet: **11.04.2012**
(21) Numéro de dépôt: 01949547.2
(22) Date de dépôt: 27.06.2001
(51) Int. Cl.: A61K 39/135, C07K 14/09, A61P 31/14

(54) **VACCIN CONTRE LA FIEVRE APHTEUSE**
MAUL- UND KLAUENSEUCHE IMPFSTOFF
VACCINE AGAINST FOOT-AND-MOUTH DISEASE

(30) Priorité: 29.06.2000 FR 0008437
(43) Date de publication de la demande: 26.03.2003
(73) Titulaire: MERIAL, 69007 Lyon (FR)
(72) Inventeur: KING, Andrew, Pirbright Woking, Surrey GU24 OJB (GB); BURMAN, Alison, Farnham, Surrey GU9 7EG (GB); AUDONNET, Jean-Christophe, F-69006 Lyon (FR); LOMBARD, Michel, F-69006 Lyon (FR)
(74) Mandataire: Nargolwalla, Cyra
(86) Numéro de dépôt international: PCT/FR2001/002042
(87) Numéro de publication internationale: WO 2002/000251

(56) Documents cités:
- WO-A-94/01133
- BEARD C ET AL: "Development of DNA vaccines for foot-and-mouth disease, evaluation of vaccines encoding replicating and non-replicating nucleic acids in swine" JOURNAL OF BIOTECHNOLOGY,NL,ELSEVIER SCIENCE PUBLISHERS, AMSTERDAM, vol. 73, no. 2-3, 20 août 1999 (1999-08-20), pages 243-249, XP004180186 ISSN: 0168-1656
- ABRAMS CHARLES C ET AL: "Assembly of foot-and-mouth disease virus empty capsids synthesized by a vaccinia virus expression system." JOURNAL OF GENERAL VIROLOGY, vol. 76, no. 12, 1995, pages 3089-3098, XP002164062 ISSN: 0022-1317 cité dans la demande
- CHINSANGARAM JARASVECH ET AL: "Antibody response in mice inoculated with DNA expressing foot-and-mouth disease virus capsid proteins." JOURNAL OF VIROLOGY, vol. 72, no. 5, mars 1998 (1998-03), pages 4454-4457, XP002164063 ISSN: 0022-538X cité dans la demande
- MAYR GREGORY A ET AL: "Development of replication-defective adenovirus serotype 5 containing the capsid and 3C protease coding regions of foot-and-mouth disease virus as a vaccine candidate." VIROLOGY, vol. 263, no. 2, 25 octobre 1999 (1999-10-25), pages 496-506, XP002164064 ISSN: 0042-6822
- FELGNER J H ET AL: "ENHANCED GENE DELIVERY AND MECHANISM STUDIES WITH A NOVEL SERIES OF CATIONIC LIPID FORMULATIONS" JOURNAL OF BIOLOGICAL CHEMISTRY,US,AMERICAN SOCIETY OF BIOLOGICAL CHEMISTS, BALTIMORE, MD, vol. 269, no. 4, 28 janvier 1994 (1994-01-28), pages 2550-2561, XP002010485 ISSN: 0021-9258
- CHO J H ET AL: "Enhanced cellular immunity to hepatitis C virus nonstructural proteins by codelivery of granulocyte macrophage-colony stimulating factor gene in intramuscular DNA immunization" VACCINE,GB,BUTTERWORTH SCIENTIFIC. GUILDFORD, vol. 17, no. 9-10, 5 mars 1999 (1999-03-05), pages 1136-1144, XP004158236 ISSN: 0264-410X
- DOEL T R ET AL: "Comparative immunogenicity of 146S, 75S and 12S particles of foot-and-mouth disease virus", ARCHIVES OF VIROLOGY, SPRINGER WIEN, AT, vol. 73, no. 2, 1 January 1982 (1982-01-01), pages 185-191, XP008081915, ISSN: 0304-8608, DOI: 10.1007/BF01314726
- LEWIS S A ET AL: "Expression, processing, and assembly of foot-and-mouth disease virus capsid structures in heterologous systems: Induction of a neutralizing antibody response in guinea pigs", JOURNAL OF VIROLOGY, THE AMERICAN SOCIETY FOR MICROBIOLOGY, US, vol. 65, no. 12, 1 December 1991 (1991-12-01), pages 6572-6580, XP002995919, ISSN: 0022-538X
- GRUBMAN M J ET AL: "Protection of swine against foot-and-mouth disease with viral capsid proteins expressed in heterologous systems", VACCINE, ELSEVIER LTD, GB, vol. 11, no. 8, 1 January 1993 (1993-01-01), pages 825-829, XP023711466, ISSN: 0264-410X, DOI: 10.1016/0264-410X(93)90357-4 [retrieved on 1993-01-01]

### LISTE DE SEQUENCES

<110> MERIAL
<120> Vaccins anti-fièvre aphteuse
<130> FMDV pseudoparticules
<140>
   <141>
<160> 41
<170> PatentIn Ver. 2.1
<210> 1
   <211> 37
   <212> ADN
   <213> Séquence artificielle
<220>
   <223> Description de la séquence artificielle: oligonucléotide
<400> 1
   ttttgatatc atgggtgctg ggcagtccag cccagca 37
<210> 2
   <211> 21
   <212> ADN
   <213> Séquence artificielle
<220>
<223> Description de la séquence artificielle: oligonucléotide.
<400> 2
   ttcacgacga aagtactatc c 21
<210> 3
<211> 21
   <212> ADN
   <213> Séquence artificielle
<220>
   <223> Description de la séquence artificielle: oligonucléotide
<400> 3
   ctgaaggacc ctactccggg c 21
<210> 4
   <211> 37
   <212> ADN
   <213> Séquence artificielle
<220>
   <223> Description de la séquence artificielle: oligonucléotide
<400> 4
   ttttagatct tcaaagcttt gttttgcgca tcacgtg 37
<210> 5
   <211> 37
   <212> ADN
   <213> Séquence artificielle
<220>
   <223> Description de la séquence artificielle: oligonucléotide
<400> 5
   ttttgatatc atgggggctg gacaatccag tccagcg 37
<210> 6
   <211> 21
   <212> ADN
   <213> Séquence artificielle
<220>
   <223> Description de la séquence artificielle: oligonucléotide
<400> 6
   ttcacgacga aggtgctgtc c 21
<210> 7
   <211> 18
   <212> ADN
   <213> Séquence artificielle
<220>
   <223> Description de la séquence artificielle: oligonucléotide
<400> 7
   aaggacccta cgccggac 18
<210> 8
   <211> 34
   <212> ADN
   <213> Séquence artificielle
<220>
   <223> Description de la séquence artificielle: oligonucléotide
<400> 8
   ttttagatct tcaaagcttg gttttgcgca tcac 34
<210> 9
   <211> 37
   <212> ADN
   <213> Séquence artificielle
<220>
   <223> Description de la séquence artificielle: oligonucléotide
<400> 9
   ttttgatatc atgggagctg ggcaatccag cccagcg 37
<210> 10
   <211> 23
   <212> ADN
   <213> Séquence artificielle
<220>
   <223> Description de la séquence artificielle: oligonucléotide
<400> 10
   ttcacgacaa acgtgctgtc cag 23
<210> 11
   <211> 21
   <212> ADN
   <213> Séquence artificielle
<220>
   <223> Description de la séquence artificielle: oligonucléotide
<400> 11
   agagcaaccg caagctgaag g 21
<210> 12
   <211> 34
   <212> ADN
   <213> Séquence artificielle
<220>
   <223> Description de la séquence artificielle: oligonucléotide
<400> 12
   ttttagatct tcaaagcttg gttttgcgca ttac 34
<210> 13
   <211> 37
   <212> ADN
   <213> Séquence artificielle
<220>
   <223> Description de la séquence artificielle: oligonucléotide
<400> 13
   ttttgatatc atgggggccg ggcaatccag tccggcg 37
<210> 14
   <211> 31
   <212> ADN
   <213> Séquence artificielle
<220> <223> Description de la séquence artificielle: oligonucléotide
<400> 14
   ttttctegag gggggccggc acgtgaaaga g 31
<210> 15
   <211> 31
   <212> ADN
   <213> Séquence artificielle
<220>
   <223> Description de la séquence artificielle: oligonucléotide
<400> 15
   ttttctcgag ggaccggtga agaagcctgt c 31
<210> 16
   <211> 37
   <212 ADN
   <213> Séquence artificielle
<220>
   <223> Description de la séquence artificielle: oligonucléotide
<400> 16
   ttttagatct tcagcggcgg aacagcgctt tgtcctc 37
<210> 17
   <211> 24
   <212> ADN
   <213> Séquence artificielle
<220>
   <223> Description de la séquence artificielle: oligonucléotide
<400> 17
   tttgacctaa cgtcggagaa gaag 24
<210> 18
   <211> 37
   <212> ADN
   <213> Séquence artificielle
<220>
   <223> Description de la séquence artificielle: oligonucléotide*
<400> 18
   ttttgaattc atgcagtcca gcccagcaac cggctcg 37
<210> 19
   <211> 44
   <212> ADN
   <213> Séquence artificielle
<220>
   <223> Description de la séquence artificielle: oligonucléotide
<400> 19
   ttttgaattc ataaaaatca aagctttgtt ttgcgcatca cgtg 44
<210> 20
   <211> 37
   <212> ADN
   <213> Séquence artificielle
<220>
   <223> Description de la séquence artificielle: oligonucléotide
<400> 20
   ttttgaattc atgggggctg gacaatccag tccagcg 37
<210> 21
   <211> 41
   <212> ADN
   <213> Séquence artificielle
<220>
   <223> Description de la séquence artificielle: oligonucléotide
<400> 21
   ttttgaattc ataaaaatca aagcttggtt ttgcgcatca c 41
<210> 22
   <211> 37
   <212> ADN
   <213> Séquence artificielle
<220>
   <223> Description de la séquence artificielle: oligonucléotide
<400> 22
   ttttgaattc atgggagctg ggcaatccag cccagcg 37
<210> 23
   <211> 41
   <212> ADN
   <213> Séquence artificielle
<220>
   <223> Description de la séquence artificielle: oligonucléotide
<400> 23
   ttttgaattc ataaaaatca aagcttggtt ttgcgcatta c 41
<210> 24
   <211> 37
   <212> ADN
   <213> Séquence artificielle
<220>
   <223> Description de la séquence artificielle: oligonucléotide
<400> 24
   ttttgaattc atgggggccg ggcaatccag tccggcg 37
<210> 25
   <211> 44
   <212> ADN
   <213> Séquence artificielle
<220>
   <223> Description de la séquence artificielle: oligonucléotide
<400> 25
   ttttgaattc ataaaaatca gcggcggaac agcgctttgt cctc 44
<210> 26
   <211> 27
   <212> ADN
   <213> Séquence artificielle
<220>
   <223> Description de la séquence artificielle: oligonucléotide
<400> 26
   tgagtccacc aggcacccga agacacc 27
<210> 27
   <211> 27
   <212> ADN
   <213> Séquence artificielle
<220>
   <223> Description de la séquence artificielle: oligonucléotide
<400> 27
   ggtgtcttcg ggtgcctggt ggactca 27
<210> 28
   <211> 36
   <212> ADN
   <213> Séquence artificielle
<220>
   <223> Description de la séquence artificielle: oligonucléotide
<400> 28
   aaagtctttg ccggcgctag ccgacactaa caaggt 36
<210> 29
   <211> 27
   <212> ADN
   <213> Séquence artificielle
<220>
   <223> Description de la séquence artificielle: oligonucléotide
<400> 29
   cgagtcagtc aggcagccgt agacacc 27
<210> 30
   <211> 27
   <212> ADN
   <213> Séquence artificielle
<220>
   <223> Description de la séquence artificielle: oligonucléotide
<400> 30
   ggtgtctacg gctgcctgac tgactcg 27
<210> 31
   <211> 36
   <212> ADN
   <213> Séquence artificielle
<220>
   <223> Description de la séquence artificielle: oligonucléotide
<400> 31
   agacgtccgt gtgttggcgc ctctggatct gtgttt 36
<210> 32
   <211> 27
   <212> ADN
   <213> Séquence artificielle
<220>
   <223> Description de la séquence artificielle: oligonucléotide
<400> 32
   tgacttgacg aggcacccgt aaacacc 27
<210> 33
   <211> 27
   <212> ADN
   <213> Séquence artificielle
<220>
   <223> Description de la séquence artificielle: oligonucléotide
<400> 33
   ggtgtttacg ggtgcctcgt aaagtca 27
<210> 34
   <211> 36
   <212> ADN
   <213> Séquence artificielle
<220>
   <223> Description de la séquence artificielle: oligonucléotide
<400> 34
   gtagtactgg gccaagccgg ccaagtaggt gtttga 36
<210> 35
   <211> 27
   <212> ADN
   <213> Séquence artificielle
<220>
   <223> Description de la séquence artificielle: oligonucléotide
<400> 35
   cgagtccacc aagcatccaa agacacc 27
<210> 36
   <211> 27
   <212> ADN
   <213> Séquence artificielle
<220>
   <223> Description de la séquence artificielle: oligonucléotide
<400> 36
   ggtgtctttg gatgcttggt ggactcg 27
<210> 37
   <211> 36
   <212> ADN
   <213> Séquence artificielle
<220>
   <223> Description de la séquence artificielle: oligonucléotide
<400> 37
   cccagggtag ttagtcctag gcgggttgta cacctt 36
<210> 38
   <211> 1148
   <212> PRT
   <213> Foot-and-mouth disease virus
<400> 38
   Met Gly Ala Gly Gln Ser Ser Pro Ala Thr Gly Ser Gln Asn Gln Ser
   1 5 10 15
   Gly Asn Thr Gly Ser Ile Ile Asn Asn Tyr Tyr Met Gln Gln Tyr Gln
   20 25 30
   Asn Ser Met Ser Thr Gln Leu Gly Asp Asn Thr Ile Ser Gly Gly Ser
   35 40 45
   Asn Glu Gly Ser Thr Asp Thr Thr Ser Thr His Thr Thr Asn Thr Gln
   50 55 60
   Asn Asn Asp Trp Phe Ser Lys Leu Ala Ser Ser Ala Phe Thr Gly Leu
   65 70 75 80
   Phe Gly Ala Leu Leu Ala Asp Lys Lys Thr Glu Glu Thr Thr Leu Leu
   85 90 95
   Glu Asp Arg Ile Leu Thr Thr Arg Asn Gly His Thr Thr Ser Thr Thr
   100 105 110
   Gln Ser Ser Val Gly Val Thr Tyr Gly Tyr Ser Thr Glu Glu Asp His
   115 120 125
   Val Ala Gly Pro Asn Thr Ser Gly Leu Glu Thr Arg Val Val Gln Ala
   130 135 140
   Glu Arg Phe Phe Lys Lys Phe Leu Phe Asp Trp Thr Thr Asp Lys Pro
   145 150 155 160
   Phe Gly Tyr Leu Thr Lys Leu Glu Leu Pro Thr Asp His His Gly Val
   165 170 175
   Phe Gly His Leu Val Asp Ser Tyr Ala Tyr Met Arg Asn Gly Trp Asp
   180 185 190
   Val Glu Val Ser Ala Val Gly Asn Gln Phe Asn Gly Gly Cys Leu Leu
   195 200 205
   Val Ala Met Val Pro Glu Trp Lys Ala Phe Asp Thr Arg Glu Lys Tyr
   210 215 220
   Gln Leu Thr Leu Phe Pro His Gln Phe Ile Ser Pro Arg Thr Asn Met
   225 230 235 240
   Thr Ala His Ile Thr Val Pro Tyr Leu Gly Val Asn Arg Tyr Asp Gln
   245 250 255
   Tyr Lys Lys His Lys Pro Trp Thr Leu Val Val Met Val Leu Ser Pro
   260 265 270
   Leu Thr Val Ser Asn Thr Ala Ala Pro Gln Ile Lys Val Tyr Ala Asn
   275 280 285
   Ile Ala Pro Thr Tyr Val His Val Ala Gly Glu Leu Pro Ser Lys Glu
   290 295 300
   Gly Ile Phe Pro Val Ala Cys Ala Asp Gly Tyr Gly Gly Leu Val Thr
   305 310 315 320
   Thr Asp Pro Lys Thr Ala Asp Pro Val Tyr Gly Lys Val Tyr Asn Pro
   325 330 335
   Pro Lys Thr Asn Tyr Pro Gly Arg Phe Thr Asn Leu Leu Asp Val Ala
   340 345 350
   Glu Ala Cys Pro Thr Phe Leu Arg Phe Asp Asp Gly Lys Pro Tyr Val
   355 360 365
   Val Thr Arg Ala Asp Asp Thr Arg Leu Leu Ala Lys Phe Asp Val Ser
   370 375 380
   Leu Ala Ala Lys His Met Ser Asn Thr Tyr Leu Ser Gly Ile Ala Gln
   385 390 395 400
   Tyr Tyr Thr Gln Tyr Ser Gly Thr Ile Asn Leu His Phe Met Phe Thr
   405 410 415
   Gly Ser Thr Asp Ser Lys Ala Arg Tyr Met Val Ala Tyr Ile Pro Pro
   420 425 430
   Gly Val Glu Thr Pro Pro Asp Thr Pro Glu Glu Ala Ala His Cys Ile
   435 440 445
   His Ala Glu Trp Asp Thr Gly Leu Asn Ser Lys Phe Thr Phe Ser Ile
   450 455 460
   Pro Tyr Val Ser Ala Ala Asp Tyr Ala Tyr Thr Ala Ser Asp Thr Ala
   465 470 475 480
   Glu Thr Thr Asn Val Gln Gly Trp Val Cys Val Tyr Gln Ile Thr His
   485 490 495
   Gly Lys Ala Glu Asn Asp Thr Leu Leu Val Ser Ala Ser Ala Gly Lys
   500 505 510
   Asp Phe Glu Leu Arg Leu Pro Ile Asp Pro Arg Thr Gln Thr Thr Thr
   515 520 525
   Thr Gly Glu Ser Ala Asp Pro Val Thr Thr Thr Val Glu Asn Tyr Gly
   530 535 540
   Gly Asp Thr Gln Val Gln Arg Arg His His Thr Asp Val Gly Phe Ile
   545 550 555 560
   Met Asp Arg Phe Val Lys Ile Asn Ser Leu Ser Pro Thr His Val Ile
   565 570 575
   Asp Leu Met Gln Thr His Lys His Gly Ile Val Gly Ala Leu Leu Arg
   580 585 590
   Ala Ala Thr Tyr Tyr Phe Ser Asp Leu Glu Ile Val Val Arg His Asp
   595 600 605
   Gly Asn Leu Thr Trp Val Pro Asn Gly Ala Pro Glu Ala Ala Leu Ser
   610 615 620
   Asn Thr Ser Asn Pro Thr Ala Tyr Asn Lys Ala Pro Phe Thr Arg Leu
   625 630 635 640
   Ala Leu Pro Tyr Thr Ala Pro His Arg Val Leu Ala Thr Val Tyr Asp
   645 650 655
   Gly Thr Asn Lys Tyr Ser Ala Ser Asp Ser Arg Ser Gly Asp Leu Gly
   660 665 670
   Ser Ile Ala Ala Arg Val Ala Thr Gln Leu Pro Ala Ser Phe Asn Tyr
   675 680 685
   Gly Ala Ile Gln Ala Gln Ala Ile His Glu Leu Leu Val Arg Met Lys
   690 695 700
   Arg Ala Glu Leu Tyr Cys Pro Arg Pro Leu Leu Ala Ile Lys Val Thr
   705 710 715 720
   Ser Gln Asp Arg Tyr Lys Gln Lys Ile Ile Ala Pro Ala Lys Gln Leu
   725 730 735
   Leu Asn Phe Asp Leu Leu Lys Leu Ala Gly Asp Val Glu Ser Asn Leu
   740 745 750
   Gly Pro Phe Phe Phe Ala Asp Val Arg Ser Asn Phe Ser Lys Leu Val
   755 760 765
   Asp Thr Ile Asn Gln Met Gln Glu Asp Met Ser Thr Lys His Gly Pro
   770 775 780
   Asp Phe Asn Arg Leu Val Ser Ala Phe Glu Glu Leu Ala Thr Gly Val
   785 790 795 800
   Lys Ala Ile Arg Thr Gly Leu Asp Glu Ala Lys Pro Trp Tyr Lys Leu
   805 810 815
   Ile Lys Leu Leu Ser Arg Leu Ser Cys Met Ala Ala Val Ala Ala Arg
   820 825 830
   Ser Lys Asp Pro Val Leu Val Ala Ile Met Leu Ala Asp Thr Gly Leu
   835 840 845
   Glu Arg Gln Lys Pro Leu Lys Val Arg Ala Lys Leu Pro Gln Gln Glu
   850 855 860
   Gly Pro Tyr Ala Gly Pro Met Glu Arg Gln Lys Pro Leu Lys Val Lys
   865 870 875 880
   Val Lys Ala Pro Val Val Lys Glu Gly Pro Tyr Glu Gly Pro Val Lys
   885 890 895
   Lys Pro Val Ala Leu Lys Val Lys Ala Arg Asn Leu Ile Val Thr Glu
               900                 905                 910
   Ser Gly Ala Pro Pro Thr Asp Leu Gln Lys Met Val Met Gly Asn Thr
           915                 920                 925
   Lys Pro Val Glu Leu Asn Leu Asp Gly Lys Thr Val Ala Ile Cys Cys
       930                 935                 940
   Ala Thr Gly Val Phe Gly Thr Ala Tyr Leu Val Pro Arg His Leu Phe
   945                 950                 955                 960
   Ala Glu Lys Tyr Asp Lys Ile Met Leu Asp Gly Arg Ala Met Thr Asp
                   965                 970                 975
   Ser Asp Tyr Arg Val Phe Glu Phe Glu Ile Lys Val Lys Arg Thr Gly
               980                 985                 990
   His Ala Leu Arg Arg Gly Thr His Trp Leu Leu His Arg Gly Asn Cys
           995                 1000                1005
   Val Arg Asp Ile Thr Lys His Phe Arg Asp Thr Ala Arg Met Lys Lys
       1010                1015                1020
   Gly Thr Pro Val Val Gly Val Val Asn Asn Ala Asp Val Gly Arg Leu
   1025                1030                1035                1040
   Ile Phe Ser Gly Glu Ala Leu Thr Tyr Lys Asp Ile Val Val Cys Met
                   1045                1050                1055
   Asp Gly Asp Thr Met Pro Gly Leu Phe Ala Tyr Lys Ala Ala Thr Arg
               1060                1065                1070
   Ala Gly Tyr Cys Gly Gly Ala Val Leu Ala Lys Asp Gly Ala Asp Thr
           1075                1080                1085
   Phe Ile Val Gly Thr His Ser Ala Gly Gly Asn Gly Val Gly Tyr Cys
       1090                1095                1100
   Ser Cys Val Ser Arg Ser Met Leu Gln Lys Met Lys Ala His Val Asp
   1105                1110                1115                1120
   Pro Glu Pro His His Glu Gly Leu Ile Val Asp Thr Arg Asp Val Glu
                   1125                1130                1135
   Glu Arg Val His Val Met Arg Lys Thr Lys Leu *
               1140                1145
<210> 39
   <211> 3444
   <212> ADN
   <213> Foot-and-mouth disease virus
<400> 39
   atgggtgctg ggcagtccag cccagcaacc ggctcgcaga accagtctgg caacactggc 60
   agcataatta acaactacta catgcagcaa taccagaact ctatgagcac acagcttggt 120
   gacaatacca tcagtggagg ctccaacgag ggctccacgg acacaacttc aacacacaca 180
   accaacaccc aaaacaacga ctggttttca aaacttgcca gttcggcttt taccggtctg 240
   ttcggtgcac ttctcgccga caagaagacg gaagagacta cgcttctgga agaccgcatc 300
   ctcactaccc gcaacgggca caccacttcg accacccagt cgagtgtggg agtcacgtat 360
   gggtactcca ctgaggaaga tcacgttgct gggcccaaca catcgggctt agagacgcgg 420
   gtggtgcagg cagagagatt tttcaagaag tttctgtttg actggacaac ggacaaacct 480
   tttggatact tgacaaaact ggagcttccc accgatcacc acggtgtctt cgggcacctg 540
   gtggactcat atgcatatat gaggaacggc tgggatgttg aggtatctgc cgtcggcaac 600
   cagttcaacg gcgggtgcct tctggtggcc atggtgccag agtggaaggc atttgacaca 660
   cgtgaaaaat accagcttac ccttttccca caccagttta ttagccccag aactaacatg 720
   actgcccaca tcacggtacc gtatcttggt gtgaacaggt acgatcagta caagaaacac 780
   aaaccttgga cactggttgt catggtacta tcacccctca cggtcagcaa cactgccgcc 840
   ccacaaatca aggtctacgc caacattgcc ccaacctacg ttcacgtggc tggagagctt 900
   ccctcgaaag aggggatttt cccagttgca tgcgcagacg gttacggagg actggtgaca 960
   acagacccga aaacagctga ccctgtttac ggtaaggtgt ataacccgcc caagaccaac 1020
   taccccgggc gctttacaaa cctattggac gtggccgaag catgtcccac ctttcttcgt 1080
   ttcgacgatg ggaaaccgta cgtcgttacg cgggcagacg acacccgtct tttggccaag 1140
   tttgatgtct cccttgccgc aaaacacatg tccaacacat acctatcagg gattgcacag 1200
   tactacacac agtactctgg tactatcaac ctgcacttca tgttcacagg ctccactgac 1260
   tcaaaagccc gctacatggt ggcttacatc ccgcctgggg tggagacgcc gccggacaca 1320
   cctgaagaag ctgctcactg cattcacgct gagtgggaca caggactgaa ctccaaattc 1380
   accttttcaa tcccttacgt gtctgccgcg gattacgcgt ataccgcatc tgatacggca 1440
   gagacaacca atgtacaggg atgggtctgt gtttaccaaa ttacacacgg gaaggctgaa 1500
   aatgacacct tgttagtgtc ggctagcgcc ggcaaagact ttgagttgcg cctcccaatt 1560
   gacccccgga cacaaaccac tactactggg gagtccgcag accctgtcac caccaccgtg 1620
   gagaactacg gcggtgatac acaagtccag agacgtcacc acacggacgt cggcttcatt 1680
   atggaccgat ttgtgaagat aaacagcctg agccccacac atgtcattga cctcatgcaa 1740
   acccacaaac acgggatcgt gggtgcgtta ctgcgtgcag ccacgtacta cttctccgac 1800
   ttggagattg ttgtgcggca cgatggtaat ctgacctggg tgcccaacgg tgcccccgag 1860
   gcagccctgt caaacaccag caaccccact gcctacaaca aggcaccgtt cacgagactt 1920
   gctctccctt acactgcgcc acaccgcgtg ttggcaactg tgtacgacgg gacaaacaag 1980
   tactccgcaa gcgattcgag atcaggcgac ctggggtcca tcgcggcgcg agtcgcgaca 2040
   caacttcctg cttcctttaa ctacggtgca atccaggcac aggccatcca cgagcttctc 2100
   gtgcgcatga aacgggccga gctctactgt cccaggccac ttctagcaat aaaggtgact 2160
   tcgcaagaca ggtacaagca aaagattatt gcgcccgcaa aacagctgtt gaactttgac 2220
   ctacttaagt tggcgggtga cgttgagtcc aaccttgggc ccttcttctt cgctgacgtt 2280
   aggtcaaact tttcgaagct ggtagacacc atcaatcaga tgcaggagga catgtccaca 2340
   aaacacggac ccgactttaa ccggttggtg tccgcttttg aggaattggc cactggggtt 2400
   aaagctatca gaaccggtct cgatgaggcc aaaccctggt acaagctcat caagctccta 2460
   agccgtctgt cgtgcatggc cgctgtggca gcacggtcca aggacccagt ccttgtggcc 2520
   atcatgctgg ccgacaccgg tctcgagcgt cagaaacctc taaaagtgag agccaagctc 2580
   ccacagcagg agggacccta cgctggcccg atggagagac agaaaccgct gaaagtaaaa 2640
   gtaaaagccc cggtcgttaa ggaaggacct tacgagggac cggtgaagaa gcctgtcgct 2700
   ttgaaagtga aagctaggaa cttgattgtc actgagagtg gtgcccccac gaccgacttg 2760
   cagaagatgg tcatgggcaa cacaaagcct gttgagctta acctcgacgg gaagacagta 2820
   gccatctgct gtgctactgg agtgttcggc actgcttacc tcgtgcctcg tcaccttttc 2880
   gcagagaagt atgacaagat tatgttggac ggcagagcca tgacagacag tgattacaga 2940
   gtgtttgagt tcgagattaa agttaaaagg acaggacatg ctctcagacg cggcactcat 3000
   tggttgcttc accgtgggaa ctgcgtgaga gacatcacga aacactttcg tgatacagca 3060
   agaatgaaga aaggcacccc cgtcgttggt gttgtcaaca acgccgatgt tgggagactg 3120
   attttctctg gtgaggccct tacctacaag gacattgtag tgtgcatgga tggagacacc 3180
   atgcccggcc tctttgccta caaagccgcc accagggctg gctactgtgg aggagccgtt 3240
   cttgccaagg acggggctga cacattcatc gtcggcactc actctgcagg tggcaatgga 3300
   gttggatact gctcatgcgt ttccaggtcc atgcttcaaa agatgaaggc tcacgtcgac 3360
   cctgaaccgc accacgaggg gttgattgtt gataccagag atgtggaaga gcgcgtccac 3420
   gtgatgcgca aaacaaagct ttga                                        3444
<210> 40
   <211> 28
   <212> ADN
   <213> Séquence artificielle
<220>
   <223> Description de la séquence artificielle: oligonucléotide
<400> 40 28
   ttttatcgat tcattgatag taccaaat 28
<210> 41
   <211> 20
   <212> ADN
   <213> Séquence artificielle
<220>
   <223> Description de la séquence artificielle: oligonucléotide
<400> 41 20
   attctacagt tctaacatcc 20

## Description

La présente invention est relative à des vaccins contre la fièvre aphteuse et en particulier à l'amélioration de leur stabilité thermique. Elle est aussi relative à des procédés de préparation de ces vaccins, à l'utilisation d'antigènes pour la réalisation de ces vaccins et les méthodes de vaccination les utilisant.

Elle est encore relative notamment à des séquences nucléotidiques, en particulier ADNc, et à des séquences en acides aminés, modifiées par rapport aux séquences naturelles du virus. Et l'invention est relative aux produits d'expression des séquences nucléotidiques modifiées et aux antigènes et virus aphteux incorporant ces modifications.

La fièvre aphteuse est l'une des maladies les plus virulentes et contagieuses touchant les animaux de rente. Cette maladie est endémique dans de nombreux pays dans le monde, notamment en Afrique, en Asie et en Amérique du Sud. En outre des foyers épidémiques apparaissent épisodiquement.

La présence de cette maladie dans un pays peut avoir des conséquences économiques très sévères par les pertes de productivité, pertes en poids et en lait dans les élevages infectés et par les embargos commerciaux imposés envers ce pays.

Les mesures contre cette maladie consistent en l'application stricte de mesures de restriction d'importation, de contrôles sanitaires et de quarantaines, à l'abattage des bêtes malades et en des programmes de vaccination à l'aide de vaccins inactivés, soit préventifs au niveau national ou régional, soit périfocal en cas d'apparition d'un foyer épidémique.

La maladie se caractérise par sa faible période d'incubation, sa nature très contagieuse, la formation d'aphtes dans la bouche et sur les pieds, et parfois par de la mortalité chez les jeunes animaux. La fièvre aphteuse touche plusieurs espèces animales, en particulier les bovins, les porcins, les ovins et les caprins.

L'agent responsable de cette maladie est un virus à acide ribonucléique (ARN) appartenant au genre des Aphthovirus et à la famille des Picornaviridae (Cooper et al., Intervirology, 1978, 10, 165-180). Le virus de la fièvre aphteuse est également désigné sous le sigle anglais FMDV (Foot-and-mouth disease virus). A ce jour, 7 types du virus de la fièvre aphteuse sont connus, les types européens (A, O et C), les types africains (SAT1, SAT2 et SAT3) et un type asiatique (Asia 1). On distingue aussi et de nombreux sous-types (Kleid et al. dans Science, 1981, 214, 1125-1129).

Il s'agit d'un virus nu de forme icosaèdrique d'environ 25 nm de diamètre renfermant une molécule d'ARN simple-brin de polarité positive d'environ 8500 nucléotides. Cette molécule d'ARN se compose d'un seul cadre ouvert de lecture (COL) qui code pour une seule polyprotéine renfermant entre autres le précurseur de la capside également nommée protéine P1 ou P88. La protéine P1 est myristylée à son extrémité amino-terminale.

Lors du processus de maturation, la protéine P1 est clivée par la protéase 3C en trois protéines nommées VP0, VP1 et VP3 (ou respectivement 1AB, 1 D et 1C) (Belsham G. J., Progress in Biophysics and Molecular Biology, 1993, 60, 241-261). Dans le virion, la protéine VP0 est ensuite clivée en deux protéines, VP4 et VP2 (ou respectivement 1A et 1B). Le mécanisme de conversion des protéines VP0 en VP1 et VP3 et de formation de virions matures n'est pas connu.

Les protéines VP1, VP2 et VP3 ont un poids moléculaire d'environ 26 000 Da, la protéine VP4 est plus petite avec environ 8 000 Da.

L'association simple des protéines de capside forme le protomère ou molécule 5S, qui est le constituant élémentaire de la capside du virus aphteux. Ce protomère se complexe ensuite en pentamère pour former la molécule 12S.

Le virion résulte de l'encapsidation d'une molécule d'ARN génomique au moyen de l'assemblage de 12 pentamères 12S, constituant ainsi les particules 146S.

La capside virale peut aussi se former sans la présence à l'intérieur de celle-ci d'une molécule d'ARN. La capside est alors vide. La capside vide est également nommée particule 70S. La formation de capsides vides peut se produire naturellement au cours de la réplication virale ou être provoquée artificiellement par traitement chimique.

Pour être efficace, un vaccin contre la fièvre aphteuse doit être en général polyvalent. Sa composition doit être renouvelé dès que le ou les types dominants sur le terrain changent: Par ailleurs pour bloquer la développement rapide de la maladie au sein du cheptel il convient d'avoir un vaccin induisant une immunité précoce et qui soit fortement protecteur.

De très nombreuses hypothèses, voies de recherche, propositions ont été faites pour tenter de développer à des vaccins efficaces contre la fièvre aphteuse. Aujourd'hui les seuls vaccins commercialisés sont des vaccins à virus inactivés.

Le traitement chimique d'inactivation peut entraîner une diminution du pouvoir immunogène du virus et de la production d'anticorps neutralisants. Les doses administrées doivent souvent être accompagnées d'adjuvants pour stimuler la réponse immunitaire, mais selon leur compositions ces adjuvants peuvent aussi induire des réactions inflammatoires. Les vaccins inactivés ne confèrent pas d'immunité sur une longue période, d'où la nécessité de pratiquer des injections de rappel tous les ans, voire plus en cas de difficultés, notamment lors d'apparitions de foyers épidémiques.

En outre il existe des risques liés à une inactivation incomplète et/ou à l'échappement de virus lors de la production de vaccins inactivés (e.g. King et al., 1981, Nature, 293, 479-480).

Le développement des techniques du génie génétique aurait dû permettre d'envisager la production de vaccins sous-unitaires et de vecteurs recombinés. Mais malgré de nombreuses tentatives, aucun d'entre eux n'est commercialisé à ce jour.

En préalable à ces travaux des études sur les capsides vides naturelles menées. Notamment Rowlands et al. (Rowlands et al., J. Gen. Virol., 1975, 26, 227-238) montrent que les virions de la fièvre aphteuse A10 comportent majoritairement les quatre protéines VP1, VP2, VP3 et VP4. Par comparaison, les capsides vides naturelles (non obtenues par recombinaison mais purifiées à partir de cultures de virus aphteux A10) contiennent essentiellement la protéine VP0 non clivée, des résultats identiques avec le virus aphteux A-Pando sont décrits par Rweyemamu (Rweyemamu et al., Archives of Virology, 1979, 59, 69-79). Les capsides vides artificielles, obtenues après dialyse en présence de Tris-EDTA et après centrifugation, ne contiennent aucune protéine VP4. Ces capsides artificielles sont, selon Rowlands *et al.*, faiblement immunogènes et les capsides vides naturelles ne sont immunogènes qu'après traitement par le formaldéhyde pour les stabiliser, la réponse en anticorps induite par les capsides vides naturelles chez le cobaye est toutefois inconstante comme le note l'auteur. En outre, Rowlands *et al.* et Rweyemamu *et al.* ne sont pas d'accord sur la nécessité de stabiliser les capsides vides naturelles. Pour Rweyemamu *et al.*, l'absence de traitement par le formaldéhyde n'est pas préjudiciable pour le niveau d'antigénicité des capsides vides naturelles. L'immunogénicité n'est testée que par l'induction d'anticorps neutralisants chez le cobaye.

Certains auteurs ne suivent pas ce courant de pensée à propos des capsides vides, voire s'y opposent. C'est le cas pour Doel T. R. et Chong W. K. T. (Doel et al., Archives of Virology, 1982, 73, 185-191) qui concluent de leurs résultats d'expérience que les capsides vides sont moins immunogènes car moins stables que les virions aphteux de sous-type A24.

L'expression du gène codant pour le précurseur P1 des protéines de capsides par un baculovirus recombiné dans des cellules d'insectes est comparée à l'expression du gène codant pour P1 associé à la protéase 3C dans *E. coli* (Grubman et al., Vaccine, 1993, 11, 825-829 ; Lewis et al., J. Virol., 1991, 65, 6572-6580). La co-expression de P1 et 3C dans *E. coli* aboutit à l'assemblage de capsides vides 70S. Le produit d'expression de ces deux constructions induit des anticorps neutralisants chez le cobaye et le porc. Les titres obtenus avec la construction P1/baculovirus sont faibles. Ces mêmes produits d'expression induisent chez le porc une protection partielle. Toutefois certains porcs protégés contre la maladie ne sont pas protégés contre la réplication du virus d'épreuve.

Les auteurs constatent par ailleurs que le système d'expression *E. coli* ne myristyle pas les protéines et que la protéase 3C est toxique pour cette cellule. Lewis et al. concluent que des questions fondamentales concernant l'assemblage du virus et la structure de la capside nécessaires pour obtenir une protection maximale chez l'animal demeurent sans réponse. Par ailleurs Grubman *et al.* précisent qu'il serait nécessaire de stabiliser les capsides vides avant de formuler le vaccin ; ils rejoignent en cela les problèmes rencontrés avec les capsides vides obtenues par extraction à partir de cultures virales (voir supra). BEARD C et al, J. Biotechnol., vol. 73, pp.243-249 décrit le développement de vaccins ADN contre la fièvre aphteuse.

Le système d'expression constitué du virus de la vaccine a aussi été utilisé pour l'obtention de capsides vides de virus aphteux A10, A24 et 01 K (Abrams et al., J. Gen. Virol., 1995, 76, 3089-3098). Principalement deux constructions ont été réalisées pour chaque sous-type, qui comportent les fragments de la séquence nucléotidique codant pour le précurseur P1 et pour la protéase 3C soit sous contrôle du promoteur précoce/tardif p7,5K du virus de la vaccine, soit du promoteur du bactériophage T7. Seules des expériences de recombinaison et d'expression *in vitro* ont été menées, sur des cultures de cellules humaines. Les cultures de cellules transformées par les constructions comportant le promoteur p7,5K n'ont pas permis l'isolement de virus de la vaccine recombinés. Les raisons en sont inconnues. Les cultures de cellules transformées par les constructions comportant le promoteur T7 ont permis l'obtention de vecteurs virus de la vaccine recombinés, l'expression des protéines virales VP0, VP1 et VP3 et l'obtention de capsides vides. Ces expériences étaient destinées à étudier la morphogénèse du virus aphteux et ne concernaient pas la production de vaccins ni l'évaluation de leur efficacité.

Des plasmides comprenant la cassette codant pour P1-2A et 3C sous la dépendance d'un promoteur hCMV-IE ont été testés par Chinsangaram *et al.* (Chinsangaram et al., J. Virol., 1998, 72(5), 4454-4457). Les injections de ces plasmides chez le porc induisent des anticorps neutralisants, et selon les groupes d'animaux une absence de protection après 2 injections ou une protection après 4 injections. Les auteurs concluent qu'il convient d'améliorer la réponse immunitaire induite par la co-expression de cytokine.

Parallèlement à ces approches, qui n'ont pas abouti à la réalisation de vaccins, d'autres auteurs se sont orientés vers l'utilisation de protéine VP1 de virus aphteux, seule ou en fusion, ou de peptides synthétiques.

Des travaux avec une protéine de fusion composée de la protéine LE de l'opéron tryptophane d'*E. coli* et d'un fragment de la protéine VP1 du virus aphteux A24 (acides aminés 131-157 sous forme de monomère) ont été menés chez le porc (Morgan et al., Am. J. Vet. Res., 1990, 51, 40-45) en comparant les résultats avec ceux obtenus avec une protéine de fusion semblable mais construite à partir du virus aphteux A12 (acides aminés 137-168 en tandem). Giavedoni *et al.* (Giavedoni et al., J. Gen. Virol., 1991, 72, 967-971) décrivent une protéine de fusion TrpE avec des fragments de la région C terminale de la protéine VP1 du virus aphteux O1. Huang *et al.* décrivent une protéine de fusion recombinée comprenant la bêta-galactosidase et deux séquences répétées en tandem de VP1 du virus aphteux O (Huang et al., Viral Immunol., 1999, 12(1), 1-8).

Des protéines du fusion comportant tout ou partie de la protéine VP1 ont aussi été obtenues par l'utilisation de vecteurs viraux, à savoir un virus herpès ou le virus de la vaccine. CA-A-2,047,585 décrit notamment un virus herpès bovin utilisé pour la production de protéines de fusion comportant une séquence peptidique du virus aphteux (acides aminés 141 à 158 de VP1 liés aux acides aminés 200 à 213 de VP1) fusionné avec la glycoprotéine gpIII de ce virus herpès bovin.

Des vaccins comportant des peptides synthétiques basés sur les régions immunogéniques de la protéine VP1 (sur ces régions, voir Strohmaier et al., J. Gen. Virol., 1982, 59, 295-306) ont également été élaborés et testés.

Agterberg et al. (Vaccine, 1990, 8, 438-440) ont produit dans des bactéries *E. coli* transformées une protéine de fusion comportant deux déterminants immunogéniques de la protéine VP1 du virus aphteux A10 (régions 141-153 et 200-207) et la protéine membranaire PhoE d'*E. coli* K-12. 100 µg de cette protéine de fusion ont ensuite été administrés par voie intramusculaire à des cobayes qui ont par la suite montré un taux d'anticorps neutralisants détectable et une protection après épreuve homologue.

WO-A-99/66954 décrit des peptides synthétiques correspondant à des séquences consensus de sites antigéniques de VP1 de virus aphteux des type A, O ou Asia (correspondant à la région 134-168 de VP1 du virus aphteux A12).

WO-A-98/12333 décrit des peptides synthétiques comprenant au moins 8 acides aminés correspondant à une séquence partielle de protéines de virus aphteux.

La présente invention a pour objectif de proposer des vaccins efficaces et sûrs contre la fièvre aphteuse.

La présente invention a également pour objectif de proposer des vaccins anti-aphteux stables.

La présente invention a aussi pour objectif de proposer de tels vaccins anti-aphteux efficaces à faible dose. L'invention telle que définie dans les revendications indépendantes.

L'invention a ainsi pour objet un vaccin contre la fièvre aphteuse, utilisant comme antigène une quantité efficace de capsides vides du virus aphteux, ces capsides vides étant obtenues in vitro par l'expression, dans des cellules eucaryotes, de l'ADN complémentaire (ADNc) de la région P1 du génome du virus aphteux codant pour la capside et de l'ADNc de la région du génome du virus aphteux codant pour la protéase 3C, le vaccin comprenant en out un véhicule ou excipient pharmaceutique acceptable sur le plan vétérinaire, dans lequel l'ADNc de la région P1 du génome du virus aphteux est modifié par remplacement du codon codant pour l'acide aminé 179 de la séquence d'Acides Aminés SED ID No. 38 ou du codon codant pour l'Acide Aminé correspondant à l'Acide Aminé 179 de la séquence D'Acides Aminés SEQ ID NO.38 par un codon codant pour une cystéine. Par définition, on n'exprime pas de protéine L fonctionnelle et par conséquent les constructions ne comprennent pas l'ADNc codant pour L, et de préférence elles ne comprennent aucun ADNc codant pour tout ou partie de L.

On préfère réaliser l'expression de P1 et d'au moins une partie de 2A, de préférence de la totalité de 2A. L'expression peut également impliquer des régions au-delà de 2A, et comprendre par exemple une partie de 2B, e.g. comme cela a été réalisé dans les exemples.

On préfère réaliser l'expression de 3C et d'au moins une partie des protéines 3B, par exemple de deux protéines 38 adjacentes et une partie non fonctionnelle de 3D, e.g. comme cela a été réalisé dans les exemples.

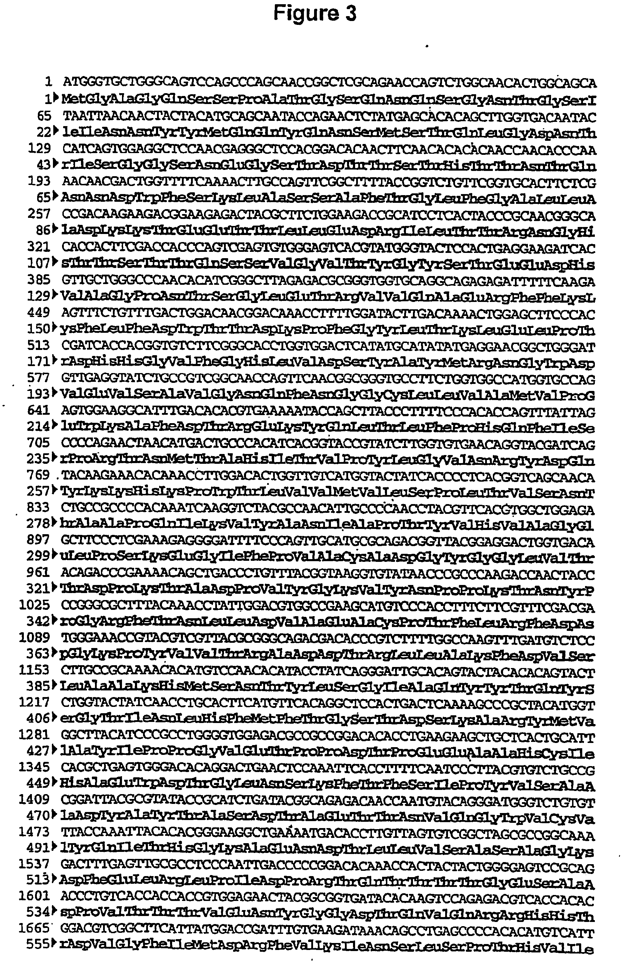

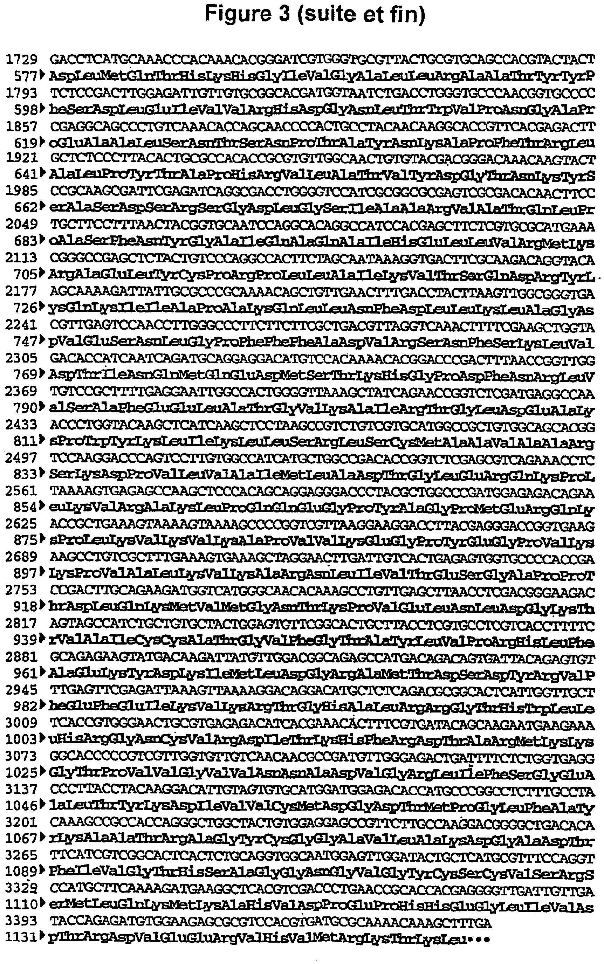

La Figure 3 (SEQ ID N° 38) donne la séquence nucléotidique et la séquence en acides aminés correspondant à P1 (acides aminés 2 à 737), 2A (acides aminés 738 à 753), 3C (acides aminés 913 à 1126) de la souche A10 du virus aphteux. Les séquences d'autres souches des différents types et sous-types sont disponibles, notamment dans Genbank ainsi que cela est mentionné dans les exemples.

Suivant une première modalité de l'invention, les capsides vides sont présentes dans le vaccin comme sous-unités et cela en quantité efficace. De préférence ces sous-unités sont obtenues par expression des ADNc des régions P1 et 3C sous la dépendance d'un promoteur, de préférence un même promoteur. De manière préférée il s'agit d'un promoteur inductible ou d'un promoteur tardif d'origine virale.

Les vecteurs d'expression utilisables comprennent notamment les vecteurs viraux, de préférence les poxvirus, notamment le virus de la vaccine, ou encore par exemple les adénovirus, les herpèsvirus et les vecteurs plasmidiques. Ces vecteurs d'expression sont utilisés pour assurer l'expression *in vitro* des capsides vides dans des cellules eucaryotes primaires ou de lignée. Une variante consiste à utiliser un vecteur d'intégration permettant d'intégrer la cassette d'expression dans la cellule eucaryote. Les cellules eucaryotes utilisables sont de préférence des cellules de lignée par exemple les cellules BHK-21, CHO, COS, RK13, Vero, MDBK, PK15.

Comme promoteur inductible on peut citer notamment le promoteur du bactériophage T7, le promoteur heat-shock, le promoteur métallothionéine, les promoteurs inductibles par l'ecdysone ou par les stéroïdes. Lorsqu'on utilise le promoteur du bactériophage T7, on réalise la co-expression de la polymérase du bactériophage T7 et des capsides vides.

Comme promoteur tardif d'origine virale on peut citer notamment, lorsqu'on utilise un poxvirus comme vecteur, le promoteur P11 K du virus de la vaccine, P28K du virus de la vaccine, P160K ATI du virus cowpox.

La conservation et le stockage des sous-unités sont de préférence assurés par congélation ou par lyophilisation.

Les doses administrées peuvent être notamment comprises entre 0,3 et 30 µg, notamment entre 0,5 et 20 µg, de préférence entre 1 et 10 µg et plus préférentiellement encore entre 1 et 5 µg, par dose.

Les volumes de dose peuvent être de préférence compris entre 0,2 et 5 ml, de préférence entre 1 et 3ml.

Le milieu de reprise (excipient, véhicule) pour les vaccins sous-unitaires est de préférence un milieu permettant la conservation des capsides vides, e.g. du type DMEM.

L'administration du vaccin sous-unitaire peut se faire notamment par la voie parentérale, de préférence par la voie sous-cutanée ou intramusculaire, ou encore la voie intradermique, notamment à l'aide d'un appareil d'administration sans aiguille (jet sous pression).

L'homme de l'art possède les compétences nécessaires pour définir précisément le nombre d'injection et les doses de chaque vaccin à utiliser pour chaque protocole de vaccination.

Ces vaccins comprennent de préférence un ou plusieurs adjuvants.

Pour adjuver les vaccins sous-unitaires selon l'invention, on peut notamment utiliser à titre d'adjuvant (1) de l'hydroxyde d'alumine, de la saponine (par exemple QuilA), de l'avridine, du DDA, (2) un polymère de l'acide acrylique ou méthacrylique, un polymère d'anhydride maléique et de dérivé alcényle, ou (3) formuler le vaccin sous forme d'une émulsion eau-dans-huile, huile-dans-eau ou eau-dans-huile-dans-eau.

L'émulsion peut notamment être à base d'huile de paraffine liquide légère (type Pharmacopée européenne) ; d'huile isoprénoide telle que le squalane ou le squalène ; d'huile résultant de l'oligomérisation d'alcènes, en particulier d'isobutène ou de décène ; des esters d'acides ou d'alcools à groupement alkyle linéaire, plus particulièrement les huiles végétales, l'oléate d'éthyle, le di(caprylate / caprate) de propylène glycol, le tri(caprylate / caprate) de glycérol, le dioléate de propylène glycol ; des esters d'acides ou d'alcools gras ramifiés, en particulier des esters de l'acide isostéarique. L'huile est utilisée en association avec des émulsifiants pour former l'émulsion. Les émulsifiants sont de préférence des tensio-actifs non ioniques en particulier les acides gras polyoxyétylénés (e.g. acide oléique), les esters de sorbitan, de mannide (e.g. oléate d'anhydromannitol), de glycérol, de polyglycérol, de propylène glycol et de l'acide oléique, isostéarique, ricinoléique, hydroxystéarique, éventuellement éthoxylés, les éthers d'alcools gras et de polyols (e.g. alcool oléique), les blocs copolymères polyoxypropylène-polyoxyéthylène en particulier les Pluronic®, notamment L121 (voir Hunter et al., 1995, "The Theory and Practical Application of Adjuvants" (Ed. Steward-Tull, D.E.S.) John Wiley and Sons, NY, 51-94 ; Todd et al., Vaccine, 1997, 15, 564-570).

Les polymères de l'acide acrylique ou méthacrylique sont réticulés notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol. Parmi eux, on peut citer les Carbopol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186: 778-780, 4 juin 1960

Sur le plan de leur structure, les polymères d'acide acrylique ou méthacrylique et les EMA® sont formés de préférence de motifs de base de formule suivante : 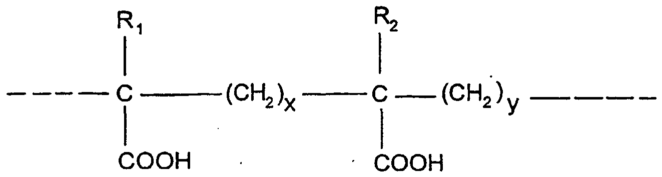 dans laquelle :
- R₁ et R₂, identiques ou différents, représentent H ou CH₃
- x = 0 ou 1, de préférence x = 1
- y=1 1 ou 2, avec x + y = 2

Pour les EMA®, x = 0 et y = 2. Pour les carbomères, x = y = 1.

La concentration en polymère dans la composition vaccinale finale sera de 0,01 % à 1,5 % P/V, plus particulièrement de 0,05 à 1 % P/V, de préférence de 0,1 à 0,4 % P/V.

Suivant une deuxième modalité de l'invention, le vaccin comprend un vecteur d'expression contenant de l'ADNc de la Region P1 du génome du virus Aphteux codant pour la capside et de l'ADNc de la Région du génome du virus Aphteux codant pour la protéase 3C, de façon à produire les capsides vides *in vivo* dans lequel l'ADNc de la région P1 du génome du virus aphteux est modifié par remplacement du codon codant pour l'acide aminé 179 DE SEQ ID NO.38 ou du codon codant pour l'Acide Aminé correspondant à L'Acide Aminé 179 de la séquence d'Acides Aminés SEQ ID NO.38 par un codon codant pour une cystéine. De préférence ces capsides vides sont obtenues *in vivo* par expression des ADNc des régions P1 et 3C insérés dans un vecteur d'expression plasmidique ou dans un vecteur d'expression viral et placés sous la dépendance d'un promoteur, de préférence d'un même promoteur. De manière préférée le promoteur est un promoteur fort précoce ou un promoteur tardif d'origine virale.

Dans le cas des vecteurs viraux on utilise de préférence un promoteur tardif d'origine virale. Les vecteurs viraux sont de préférences les poxvirus, notamment le virus de la vaccine, les avipox (e.g. fowlpox, canarypox), le racoonpox, le swinepox, le capripox, ou encore les adénovirus réplicatifs, notamment l'adénovirus porcin, et les herpèsvirus. Comme promoteur tardif d'origine virale on peut citer notamment, pour les poxvirus, le promoteur P11K du virus de la vaccine, P28K du virus de la vaccine, P160K ATI du virus cowpox.

Par définition, un vecteur d'expression plasmidique (ou plasmide) recouvre une unité de transcription ADN comprenant une séquence polynucléotidique comprenant l'ADNc à exprimer et les éléments nécessaires à son expression *in vivo.* On préfère la forme plasmide circulaire, super-enroulée ou non. La forme linéaire entre également dans le cadre de cette invention.

Dans le cas des plasmides on utilise de préférence un promoteur fort précoce d'origine virale ou d'origine cellulaire et en particulier le promoteur précoce du cytomégalovirus CMV-IE, d'origine humaine ou murine, ou encore éventuellement d'une autre origine telle que rat, cobaye. On peut également utiliser le promoteur précoce ou tardif du virus SV40 ou le promoteur LTR du virus du Sarcome de Rous. Comme promoteur cellulaire, on peut citer le promoteur d'un gène du cytosquelette, tel que par exemple le promoteur de la desmine, ou encore le promoteur de l'actine.

La conservation et le stockage des vaccins recombinés sont de préférence assurés par congélation ou par lyophilisation ou sous forme liquide.

Le milieu de reprise (excipient, véhicule) est de préférence une solution saline NaCl à 0,9 % ou un tampon phosphate.

La quantité de vecteurs viraux utilisée dans les vaccin selon la présente invention est d'environ au moins 10³ pfu. Elle est de préférence comprise entre environ 10⁴ pfu et environ 10¹⁰ pfu, e.g. environ 10⁵ pfu et 10⁹ pfu, plus particulièrement entre environ 10⁶ pfu et environ 10⁸ pfu, par dose.

La quantité de vecteurs plasmidiques utilisée dans les vaccins selon la présente invention est comprise entre environ 1 µg et environ 2 mg, et préférentiellement entre environ 50 µg et environ 1 mg, par dose.

Les volumes de dose peuvent être de préférence compris entre 0,2 et 5 ml, de préférence entre 1 et 3 ml.

Les vaccins recombinés selon l'invention peuvent être administrés, par les différentes voies d'administration usuelles et au moyen des techniques

d'administration connues. Suivant une modalité préférée de l'invention, Les vaccins sont formulés pour administration par la voie intramusculaire, sous-cutanée ou à l'aide d'un injecteur sans aiguille par voie intradermique. En particulier pour les vecteurs viraux on préfère la voie intramusculaire ou sous-cutanée. Pour les vecteurs viraux ou plasmidiques on peut également utiliser la voie mucosale (e.g. orale, nasale).

De préférence, ces vaccins comprennent un ou plusieurs adjuvants. Pour les vecteurs viraux on utilise avantageusement un polymère de l'acide acrylique ou méthacrylique, ou un polymère d'anhydride maléique et de dérivé alcényle, et en particulier les carbomères, notamment Carbopol® (ces adjuvants sont décrits supra).

Pour les vecteurs plasmidiques, il est avantageux de les formuler par addition à titre d'adjuvant, de lipides cationiques contenant un sel d'ammonium quaternaire, de formule : 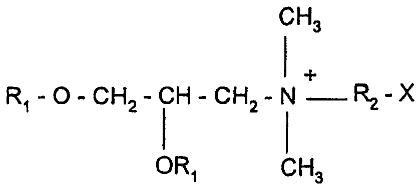 dans laquelle R1 est un radical aliphatique linéaire, saturé ou insaturé, ayant de 12 à 18 atomes de carbone, R2 est un autre radical aliphatique, renfermant 2 ou 3 atomes de carbone, et X un groupement hydroxyle ou amine.

De préférence il s'agit du DMRIE (N-(2-hydroxyéthyl)-N,N-diméthyl-2,3-bis(tetradécyloxy)-1-propanammonium ; WO-A-9634109), de préférence associé avec un lipide neutre, notamment le DOPE (dioléoyl-phosphatidyl-éthanolamine ; Behr J.P., 1994, Bioconjugate Chemistry, 5, 382-389) pour former le DMRIE-DOPE. De préférence, le mélange plasmide avec cet adjuvant se fait de manière extemporanée et l'on préfère, avant son administration à l'animal, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, et est plus particulièrement de 1 : 1.

Le ratio pondéral plasmide : adjuvant, notamment DMRIE ou DMRIE-DOPE peut aller notamment de 50 : 1 à 1 : 10, en particulier de 10 : 1 à 1 : 5, et de préférence de 1 : 1 à 1 : 2.

Les vaccins selon l'invention, adjuvés ou non comme décrit ci-dessus, peuvent encore être adjuvés par une ou des cytokines, ajoutées ou exprimées *in vivo.* De préférence, on utilise le GM-CSF (en anglais Granulocyte Macrophage Colony Stimulating Factor : Clark S.C. et al. Science 1987. 230. 1229 ; Grant S. M. et al. Drugs 1992. 53. 516), ce qui peut se faire par l'incorporation de protéine GM-CSF directement dans la composition vaccinale ou de préférence par l'insertion de la séquence nucléotidique codant pour le GM-CSF dans un vecteur d'expression dans des conditions permettant son expression *in vivo,* e.g. le vecteur contenant la séquence nucléotidique codant pour l'antigène FMDV ou un autre vecteur. Comme vecteur d'expression, on préfère utiliser un plasmide. Le choix du GM-CSF se fait de préférence en fonction de l'espèce animale à vacciner ; ainsi pour les bovins le GM-CSF bovin (voir e.g. Maliszewski et al. Molec. Immunol. 1988. 25. 843-850) est utilisé ; pour les porcs il s'agit du GM-CSF porcin (voir e.g. Inumaru S. et Takamatsu H., Immunol. Cell. Biol. 1995. 75. 474-476).

Pour la réalisation de vecteurs exprimant le GM-CSF, les séquences du GM-CSF sont disponibles dans Genbank, D21074 pour le porc, U22385 pour le bovin et X55991 pour l'ovin.

Le présent mémoire décrit un procédé de préparation d'un vaccin anti-aphteux sous-unitaire dans lequel on produit des capsides vides de ce virus par expression de l'ADNc de la région P1 et par expression de l'ADNc de la région 3C, et on les formule dans un véhicule ou excipient acceptable au plan vétérinaire, de préférence en présence d'au moins un adjuvant.

L'invention a aussi pour objet l'utilisation de capsides vides du virus Aphteux produites *in vitro* par l'expression dans des cellules eucaryotes, de l'ADNc de la région P1 du génome du virus aphteux codant pour la capside et de l'ADNc de la région du génome du virus aphteux codant pour la protéase 3C, dans lequel l'ADNC de la région P1 du génome du virus aphteux est modifié par remplacement du codon codant pour l'acide aminé 179 De la séquence d'Acides Aminés SEQ ID NO.38, ou du codon codant pour l'Acide Aminé correspondant à l'Acide Aminé 179 de la séquence d'Acides Aminés SEQ ID No.38 par un codon codant pour une cystéine, pour la préparation d'un vaccin anti-aphteux sous-unitaire, comprenant en outre un véhicule ou excipient acceptable au plan vétérinaire, de préférence en présence d'au moins un adjuvant. Est décrite une méthode de vaccination anti-aphteuse, comprenant l'administration à l'animal, notamment les animaux de rente, en particulier bovin, ovin, porcin, caprin, d'un vaccin sous-unitaire conforme à l'invention. Modes d'administration et doses ont été définis plus haut.

Les différentes caractéristiques décrites plus haut à propos du vaccin sous-unitaire selon l'invention s'appliquent à ces différents objets.

Le mémoire décrit un procédé de préparation d'un vaccin anti-aphteux recombiné dans lequel on produit des vecteurs viraux ou plasmidiques exprimant *in vivo* la protéine P1 et la protéine 3C dans des conditions conduisant à la formation de capsides vides, et on formule ces vecteurs dans un véhicule ou excipient acceptable au plan vétérinaire, de préférence en présence d'au moins un adjuvant.

L'invention a aussi pour objet l'utilisation de vecteurs viraux ou plasmidiques conformes à l'invention, pour la préparation d'un vaccin anti-aphteux recombiné, comprenant en outre un véhicule ou excipient acceptable au plan vétérinaire, de préférence en présence d'au moins un adjuvant.

Est décrite une méthode de vaccination anti-aphteuse, comprenant l'administration à l'animal, notamment les animaux de rente, en particulier bovin, ovin, porcin, caprin, d'un vaccin recombiné conforme à l'invention. Modes d'administration et doses ont été définis plus haut.

Les différentes caractéristiques décrites plus haut à propos des vaccins utilisant des vecteurs viraux ou plamidiques selon l'invention s'appliquent à ces différents objets.

Est aussi décrit un vaccin multivalent ou une association de vaccins comprenant une vaccin conforme à l'invention, et au moins un autre vaccin contre un virus aphteux d'un autre type (e.g. O, A, C, SAT1, SAT2, SAT3, Asia), d'un autre sous-type (e.g. A10, A12, A24, O1) ou d'un autre variant, de préférence conforme à l'invention, dans un véhicule ou excipient acceptable sur le plan vétérinaire et de préférence avec un adjuvant, notamment l'un de ceux décrits précédemment.

Est également décrit pour un vaccin multivalent ou une association de vaccins comprenant un vaccin conforme à l'invention, et au moins un vaccin contre un autre pathogène, notamment le virus de la rage, dans un véhicule ou excipient acceptable sur le plan vétérinaire et de préférence avec un adjuvant, notamment l'un de ceux décrits précédemment.

L'invention a pour objet l'amélioration de la stabilité à la température des capsides vides de virus aphteux et des vaccins obtenus.

L'amélioration de la stabilité à la température des capsides vides est assurée avantageusement par la formation de ponts disulfures,

cette amélioration étant obtenue par le remplacement d'un acide aminé de la séquence d'origine par un acide aminé cystéine dans la séquence polypeptidique d'une protéine de structure de la capside, la protéine VP2, cet acide aminé étant en position 179 sur la séquence d'acides aminés SEQ ID N° 38 (Figure 3). En règle générale, la position de cet acide aminé est identique chez les autres virus aphteux (c'est notamment le cas des souches décrites dans les exemples). Sur les séquences d'autres virus, la position peut éventuellement être très légèrement différente et par exemple être 178 ou 180. La région qui comprend cet acide aminé correspond à une hélice alpha. Pour identifier ou confirmer l'acide aminé à muter, on aligne les séquences en acides aminés de cette région avec la région correspondante (par exemple de l'ordre d'une dizaine ou un peu plus - e.g. 10 à 20 - d'acides aminés) sur la séquence SEQ ID N° 38, compte tenu du fait que les séquences sont bien conservées dans leur structure parmi les différents virus aphteux. On a notamment constaté en comparant les séquences des souches O1, A10, A24, A22, C1, C3, SAT2, que la région peut s'écrire de la manière suivante :
X₁ Gly X₃ X₄ Gly X₆ Leu X₈ X₉ Ser X₁₁ X₁₂ Tyr Met
   avec
X₄ et X₁₁ sont Tyr, His ou Phe (acides aminés hydrophobes)
X₃, X₈ et X₁₂ sont Val, Met, Ile, Thr ou Ala
X₆ est His, Gin, Arg, Lys, Ser ou Gly ; c'est l'acide aminé à muter en Cys
X₁ est His ou Lys (acides aminés basiques)
X₉ est Asp, Glu ou Lys (acides aminés acide et basique).

L'acide aminé à muter est l'histidine située en position 179 du précurseur P1 du virus aphteux A10.

Il s'agit de la sérine située en position 179 du précurseur P1 du virus aphteux O1.

Il s'agit de la glycine située en position 179 du précurseur P1 du virus aphteux C1.

Il s'agit de l'histidine située en position 179 du précurseur P1 du virus aphteux A24.

Par convention, la méthionine correspondant au codon d'initiation (qui n'est pas présent dans la séquence naturelle et est donc ajouté) est numérotée 1

Au niveau nucléotide, cela revient à remplacer les codons d'origine par un codon codant pour la cystéine, soit un codon TGT ou TGC pour l'ADNc, ou UGU ou UGC pour l'ARN.

La présente invention décrit les séquences nucléotidiques notamment les ADNc incorporant cette modification. En particulier, l'invention décrit les séquences ADNc, et les vecteurs les incorporant, comprenant la séquence codant pour VP2 (ou VP0), et plus particulièrement pour P1, qui incorporent cette modification, par exemple séquences ADNc codant pour P1-2A ou P1-2A-partie de 2B, et les séquences les incorporant, par exemple séquences les incorporant avec les séquences permettant leur expression (promoteur, codon ATG, etc.).

La présente invention décrit les séquences en acides aminés, obtenues à partir dé ces séquences nucléotidiques, ainsi que les capsides vides et les virus aphteux thermostables (c'est-à-dire ayant une stabilité thermique améliorée). Ils comprennent des ponts disulfure qui ne sont pas présents dans les capsides et virus naturels. En particulier, ils comprennent des protéines VP2 comprenant une cystéine à la place d'un acide aminé naturel, comme cela vient d'être décrit.

Les vaccins décrits supra sont basés sur l'utilisation de cette modification et donc les séquences d'ADNc sont modifiées en conséquence et les capsides vides obtenus soit *in vitro* soit *in vivo* possèdent les ponts disulfures.

De même, tous les autres objets (méthodes, utilisation, procédés) de l'invention qui ont été décrits supra reprennent ces caractéristiques.

L'invention va être maintenant décrite plus en détail à l'aide de modes de réalisation pris à titre d'exemples non limitatifs et se référant aux dessins dans lesquels :
**Figure 1** **:** graphe des titres en anticorps neutralisants anti-aphteux A24 mesurés sur bovins (exprimés en log)
**Figure 2** **:** photographie des gels obtenus
**Figure 3** **:** séquence nucléotidique et séquence en acides aminés correspondant à P1 (acides aminés 2 à 737), 2A (acides aminés 738 à 753), 3C (acides aminés 913 à 1126) de la souche A10 du virus aphteux

### Listes des séquences SEQ ID pour les constructions de la présente invention

**SEQ ID N° 1 :** oligonucléotide JCA305
**SEQ ID N° 2 :** oligonucléotide JCA306
**SEQ ID N° 3 :** oligonucléotide JCA307
**SEQ ID N° 4 :** oligonucléotide JCA308
**SEQ ID N° 5 :** oligonucléotide JCA309
**SEQ ID N° 6 :** oligonucléotide JCA310
**SEQ ID N° 7 :** oligonucléotide JCA311
**SEQ ID N° 8 :** oligonucléotide JCA312
**SEQ ID N° 9 :** oligonucléotide JCA313
**SEQ ID N° 10 :** oligonucléotide JCA314
**SEQ ID N° 11 :** oligonucléotide JCA315
**SEQ ID N° 12 :** oligonucléotide JCA316
**SEQ ID N° 13 :** oligonucléotide JCA317
**SEQ ID N° 14 :** oligonucléotide JCA318
**SEQ ID N° 15 :** oligonucléotide JCA319
**SEQ ID N° 16 :** oligonucléotide JCA320
**SEQ ID N° 17 :** oligonucléotide JCA321
**SEQ ID N° 18 :** oligonucléotide JCA322
**SEQ ID N° 19 :** oligonucléotide JCA323
**SEQ ID N° 20 :** oligonucléotide JCA324
**SEQ ID N° 21 :** oligonucléotide JCA325
**SEQ ID N° 22 :** oligonucléotide JCA326
**SEQ ID N° 23 :** oligonucléotide JCA327
**SEQ ID N° 24 :** oligonucléotide JCA328
**SEQ ID N° 25 :** oligonucléotide JCA329
**SEQ ID N° 26 :** oligonucléotide JCA330
**SEQ ID N° 27 :** oligonucléotide JCA331
**SEQ ID N° 28 :** oligonucléotide JCA332
**SEQ ID N° 29 :** oligonucléotide JCA333
**SEQ ID N° 30 :** oligonucléotide JCA334
**SEQ ID N° 31 :** oligonucléotide JCA335
**SEQ ID N° 32 :** oligonucléotide JCA336
**SEQ ID N° 33 :** oligonucléotide JCA337
**SEQ ID N° 34 :** oligonucléotide JCA338
**SEQ ID N° 35 :** oligonucléotide JCA339
**SEQ ID N° 36 :** oligonucléotide JCA340
**SEQ ID N° 37:** oligonucléotide JCA341
**SEQ ID N° 38 :** séquence en acides aminés correspondant à P1 (acides aminés 2 à 737), 2A (acides aminés 738 à 753), 3C (acides aminés 913 à 1126) du virus aphteux A10.
**SEQ ID N° 39 :** séquence nucléotidique correspondant à P1, 2A, 3C du virus aphteux A10.
**SEQ ID N° 40 :** oligonucléotide JCA342
**SEQ ID N° 41 :** oligonucléotide JCA343

### Exemples :

Toutes les constructions sont réalisées en utilisant les techniques standards de biologie moléculaire (clonage, digestion par les enzymes de restriction, synthèse d'un ADN complémentaire simple brin, amplification en chaîne par polymérase, élongation d'un oligonucléotide par une ADN polymérase...) décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention, ainsi que les divers fragments d'amplification en chaîne par polymérase (= ACP ou PCR), sont isolés et purifiés en utilisant le kit "Geneclean®" (BIO101 Inc. La Jolla, CA).

Des séquences nucléotidiques et polypeptidiques de virus de la fièvre aphteuse sont accessibles auprès de la base de donnée GenBank, notamment sous les numéros X00429 pour A10, X00871 pour O1K, AJ251476 pour A24, et AJ133357 pour C Spain Olot. Les exemples 1 A 14 ci-dessous décrivent des Techniques standards.

### Exemple 1 : Culture des souches de virus aphteux

Pour leur amplification, les souches de virus aphteux désignées O1K (Forss et al., 1984, Nucleic Acids Res., 12(16), 6587-6601), A24 (Weddell et al., 1985, Proc. Natl. Acad. Sci. USA, 82, 2618-2622), A10 (Carroll et al., 1984, Nucleic Acids Res., 12(5), 2461-2472), C Spain Olot (Toja et al., 1999, Virus Res., 64(2), 161-171) sont cultivées sur cellules BHK-21 (Baby Hamster Kidney, accessible auprès de l'American Type Culture Collection (ATCC) sous le numéro CCL-10). Les cellules BHK-21 sont mises en culture en Falcon 25 cm² avec du milieu Eagle-MEM supplémenté de 1 % d'extraits de levure et de 5 % de sérum de veau contenant environ 100 000 cellules par ml. Les cellules sont cultivées à +37°C.

Après 3 jours la couche cellulaire arrive à confluence. Le milieu de culture est alors remplacé par du milieu Eagle-MEM sans sérum mais supplémenté avec 0,4 % d'hydrolysat de lactalbumine et 0,3 % de peptone (pH 7,4) et le virus aphteux est ajouté à raison de 1 pfu pour environ 20 cellules.

Lorsque l'effet cytopathogène (CPE) est complet (généralement 24 heures après le début de la mise en culture), les suspensions virales sont récoltées, puis clarifiées par centrifugation et congelées à -70°C. 3 à 4 passages successifs sont généralement nécessaires à la production d'un lot viral. Le lot viral est stocké à -70°C.

Ces opérations sont renouvelées pour chacune des souches de virus aphteux.

### Exemple 2 : Extraction de l'ARN viral des souches virus aphteux

L'ARN viral contenu dans 100 ml de suspension virale de la souche de virus aphteux A24, obtenue à l'exemple 1, est extrait après décongélation avec les solutions du kit « High Pure^{™} Viral RNA Kit » Cat # 1 858 882, Roche Molecular Biochemicals), en suivant les instructions du fournisseur pour les étapes d'extraction. Le culot d'ARN obtenu à la fin de l'extraction est re-suspendu avec 10 ml d'eau distillée stérile sans RNase.

L'ARN viral de chacune des souches de virus aphteux est extrait dans les mêmes conditions.

### Exemple 3 : Construction du plasmide d'expression pour les capsides vides du virus aphteux A10

L'ADN complémentaire (ADNc) du virus aphteux A10 est synthétisé avec le kit « Gene Amp RNA PCR Kit » (Cat # N 808 0017, Perkin-Elmer, Norwalk, CT 06859, USA) en utilisant les conditions données par le fournisseur. Pour le premier fragment, une réaction de transcription inverse, suivie d'une réaction d'amplification en chaîne (Réaction « TI-ACP » ou « RT-PCR ») est réalisée avec 50 µl de la suspension d'ARN viral du virus aphteux A10 (exemple 2) et avec les oligonucléotides suivants :
JCA305 (37 mer) (SEQ ID NO 1)
5' TTTTGATATCATGGGTGCTGGGCAGTCCAGCCCAGCA 3'
et JCA306 (21 mer) (SEQ ID NO 2)
5' TTCACGACGAAAGTACTATCC 3'.

Ce couple d'oligonucléotides permet l'incorporation d'un site de restriction EcoRV et d'un codon initiateur ATG en phase avec la séquence nucléotidique codant pour P1.

La synthèse du premier brin d'ADNc se fait par élongation de l'oligonucléotide JCA306, après hybridation de ce dernier à la matrice d'ARN.

Les conditions de synthèse du premier brin d'ADNc sont une température de 42°C pendant 15 min, puis 99°C pendant 5 min, et enfin 4°C pendant 5 min. Les conditions de la réaction ACP en présence du couple d'oligonucléotides JCA305 et JCA306 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 2583 pb.

Ce fragment est digéré par EcoRV puis par XhoI pour isoler, après électrophorèse en gel d'agarose, le fragment EcoRV-XhoI d'environ 2550 pb. Ce fragment est appelé fragment A.

Pour le deuxième fragment, une réaction d'ACP est réalisée avec 50 µl de la suspension d'ARN viral du virus aphteux A10 (exemple 2) et avec les oligonucléotides suivants :
JCA307 (21 mer) (SEQ ID NO 3)
5' CTGAAGGACCCTACTCCGGGC 3'
et JCA308 (37 mer) (SEQ ID NO 4)
5' TTTTAGATCTTCAAAGCTTTGTTTTGCGCATCACGTG 3'.

Ce couple d'oligonucléotides permet l'incorporation d'un site de restriction BgIII et d'un codon stop en phase avec la séquence nucléotidique codant pour la protéase 3C.

La synthèse du premier brin d'ADNc se fait par élongation de l'oligonucléotide JCA308, après hybridation de ce dernier à la matrice d'ARN.

Les conditions de synthèse du premier brin d'ADNc sont une température de 42°C pendant 15 min, puis 99°C pendant 5 min, et enfin 4°C pendant 5 min. Les conditions de la réaction ACP en présence du couple d'oligonucléotides JCA307 et JCA308 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 936 pb.

Ce fragment est digéré par BgIII puis par XhoI pour isoler, après électrophorèse en gel d'agarose, le fragment BgIII-XhoI d'environ 900 pb. Ce fragment est appelé fragment B.

Le fragment A et le fragment B sont ligaturés avec le plasmide d'expression pVR1012 (Hartikka J. et al., 1997, Human Gene Therapy, 7, 1205-1217), préalablement digéré par BgIII et EcoRV, pour donner le plasmide pJCA161 (8327 pb). Ce plasmide contient, sous le contrôle du promoteur précoce du cytomégalovirus humain ou hCMV-IE (human Cytomegalovirus Immediate Early), un insert codant pour la partie de la polyprotéine suffisante pour générer des protéines de capside capables de s'auto-assembler.

### Exemple 4 : Construction des plasmide d'expression pour les capsides vides du virus aphteux des sous-types 01 K, C Spain Olot et A24

Les ADNc des virus aphteux des sous-types 01 K, C Spain Olot et A24 sont synthétisés comme cela est décrit dans l'exemple 3.

Pour O1K, le couple d'oligonucléotide :
JCA309 (37 mer) (SEQ ID NO 5)
5' TTTTGATATCATGGGGGCTGGACAATCCAGTCCAGCG 3'
et JCA310 (21 mer) (SEQ ID NO 6)
5' TTCACGACGAAGGTGCTGTCC 3'
est utilisé lors de la première réaction d'ACP pour produire un fragment de 2583 paires de bases (pb), puis après digestion et isolement un fragment EcoRV-XhoI d'environ 2550 pb. Ce fragment est appelé fragment C.

Lors de la deuxième réaction d'ACP, le couple d'oligonucléotides :
JCA311 (18 mer) (SEQ ID NO 7)
5' AAGGACCCTACGCCGGAC 3'
et JCA312 (34 mer) (SEQ ID NO 8)
5' TTTTAGATCTTCAAAGCTTGGTTTTGCGCATCAC 3'
est utilisé pour produire un fragment de 930 pb, puis après digestion et isolement un fragment BgIII-XhoI d'environ 900 pb. Ce fragment est appelé fragment D.

Le fragment C et le fragment D sont ligaturés avec le plasmide d'expression pVR1012, préalablement digéré par BgIII et EcoRV, pour donner le plasmide pJCA162 (8327 pb).

Pour C Spain Olot, le couple d'oligonucléotide :
JCA313 (37 mer) (SEQ ID NO 9)
5' TTTTGATATCATGGGAGCTGGGCAATCCAGCCCAGCG 3'
et JCA314 (23 mer) (SEQ ID NO 10)
5' TTCACGACAAACGTGCTGTCCAG 3',
est utilisé lors de la première réaction d'ACP pour produire un fragment de 2568 paires de bases (pb), puis après digestion et isolement un fragment EcoRV-Xhol d'environ 2540 pb. Ce fragment est appelé fragment E.

Lors de la deuxième réaction d'ACP, le couple d'oligonucléotides :
JCA315 (21 mer) (SEQ ID NO 11)
5' AGAGCAACCGCAAGCTGAAGG 3'
et JCA316 (34 mer) (SEQ ID NO 12)
5' TTTTAGATCTTCAAAGCTTGGTTTTGCGCATTAC 3',
est utilisé pour produire un fragment de 947 pb, puis après digestion et isolement un fragment BgIII3-XhoI d'environ 900 pb. Ce fragment est appelé fragment F.

Le fragment E et le fragment F sont ligaturés avec le plasmide d'expression pVR1012, préalablement digéré par BgIII et EcoRV, pour donner le plasmide pJCA163 (8312 pb).

Pour A24, le couple d'oligonucléotide :
JCA317 (37 mer) (SEQ ID NO 13)
5' TTTTGATATCATGGGGGCCGGGCAATCCAGTCCGGCG 3'
et JCA318 (31 mer) (SEQ ID NO 14)
5' TTTTCTCGAGGGGGGCCGGCACGTGAAAGAG 3',
est utilisé lors de la première réaction d'ACP pour produire un fragment d'environ 2630 paires de bases (pb), puis après digestion et isolement un fragment EcoRV-Xhol d'environ 2580 pb. Ce fragment est appelé fragment G.

Lors de la deuxième réaction d'ACP, le couple d'oligonucléotides :
JCA319 (31 mer) (SEQ ID NO 15)
5' TTTTCTCGAGGGACCGGTGAAGAAGCCTGTC 3'
et JCA320 (37 mer) (SEQ ID NO 16)
5' TTTTAGATCTTCAGCGGCGGAACAGCGCTTTGTCCTC 3'.
est utilisé pour produire un fragment d'environ 950 pb, puis après digestion et isolement un fragment BgIII-XhoI d'environ 940 pb. Ce fragment est appelé fragment H.

Le fragment G et le fragment H sont ligaturés avec le plasmide d'expression pVR1012, préalablement digéré par BgIII et EcoRV, pour donner le plasmide pJCA164 (d'une taille d'environ 8400pb).

### Exemple 5 : Construction du plasmide d'expression pour A24/A10

L'ADNc du virus aphteux A24 est synthétisé comme cela est décrit dans l'exemple 3.

Une réaction d'ACP est réalisée avec 50 µl de la suspension d'ARN de virus aphteux A24 (exemple 2) et avec les oligonucléotides suivants :
JCA317 (37 mer) (SEQ ID NO 13)
et JCA321 (24 mer) (SEQ ID NO 17)
5' TTTGACCTAACGTCGGAGAAGAAG 3'.
Un fragment d'environ 2300 pb est produit.

Ce fragment est ensuite digéré par EcoRV puis par HindIII pour isoler, après électrophorèse en gel d'agarose, le fragment EcoRV-HindIII d'environ 1710 Ce fragment est appelé fragment 1.

Le fragment de 2300 pb est digéré par HindIII puis par ApaI pour isoler, après électrophorèse en gel d'agarose, le fragment HindIII-ApaI d'environ 550 pb. Ce fragment est appelé fragment J.

Le plasmide pJCA161 (exemple 3) est digéré par ApaI puis par EcoRV pour isoler, après électrophorèse en gel d'agarose, le fragment ApaI-EcoRV d'environ 5960 pb. Ce fragment est appelé fragment K.

Les fragments I, J et K sont ligaturés ensemble pour donner le plasmide pJCA165 (8333 pb). Ce plasmide contient un insert codant pour la partie structurale de la polyprotéine A24 et pour la partie enzymatique de A10, parties suffisantes pour générer des protéines de capside capables de s'auto-assembler.

### Exemple 6 : Construction du virus recombiné vaccine vV100 (A10)

Une réaction ACP est réalisée en utilisant le plasmide pJCA161 (exemple 3) comme matrice et les oligonucléotides suivants :
JCA322 (37 mer) (SEQ ID NO 18) :
5' TTTTGAATTCATGCAGTCCAGCCCAGCAACCGGCTCG 3'
et JCA323 (44 mer) (SEQ ID NO 19) :
5' TTTTGAATTCATAAAAATCAAAGCTTTGTTTTGCGCATCACGTG 3'
pour amplifier un fragment d'environ 3462 pb.

Ce couple d'oligonucléotides permet l'incorporation d'un site de restriction EcoRI à chaque extrémité du fragment d'amplification.

Les conditions de la réaction d'ACP en présence de ce couple d'oligonucléotides sont une température de 95°C pendant 2 min, puis 35 cycles (95°C pendant 1 min, puis 62°C pendant 1 min, et 72°C pendant 2 min), et enfin 72°C pendant 7 min.

Ce fragment est digéré par EcoRI pour isoler, après électrophorèse en gel d'agarose, le fragment EcoRI-EcoRI de 3448 pb.

Une réaction d'ACP sur génome du virus de la vaccine (souche WR) est réalisée avec le couple d'oligonucléotides :
JCA342 (28 mer) SEQ ID N° 40
5' TTTTATCGATTCATTGATAGTACCAAAT 3'
JCA343 (20 mer) SEQ ID N° 41
5' ATTCTACAGTTCTAACATCG 3'.

Les conditions d'ACP sont les mêmes que précédemment. Un fragment de 488 pb est produit. Ce fragment est digéré par ClaI et EcoRI, pour isoler, après électrophorèse en gel d'agarose, le fragment de 367 pb. Ce dernier fragment est ensuite inséré dans le plasmide pGS20 (Mackett et al., J ; Virol., 1984, 49, 857-864), préalablement digéré par ClaI et EcoRI. Le plasmide ainsi obtenu est linéarisé par EcoRI et le fragment EcoRI-EcoRI de 3448 pb y est inséré, donnant le vecteur pJCA166.

Avantageusement, l'homme de l'art peut aussi se servir du plasmide pvFOHC (Newton et al., in : Vaccines 87, 1987, Chanock et al. eds., Cold Spring Harbor Laboratory, 12-21) comportant le promoteur P11K du virus de la vaccine et deux bras du gène TK du virus de la vaccine, l'un en amont de ce promoteur et l'autre en aval d'un site EcoRI.

Des sites d'insertion dans le virus de la vaccine autre que le gène TK peuvent être utilisés, notamment par exemple le gène HA, M₂L.

Le plasmide pJCA166 est transfecté dans des cellules COS (cellules rénales de singes verts africains, déposées auprès de l'American Type Culture Collection sous le numéro d'accès ATCC CRL-1651) infectées avec du virus de la vaccine (souche WR, numéro ATCC VR-119).

Les cellules COS sont cultivées en boîtes de Petri en milieu de culture DMEM (Dulbecco's Modified Eagles Medium) supplémenté de 10 % de sérum de veau foetal, 2 mM du glutamine, 500 Ul/µg/ml de penicilline/streptomycine, 12,5 µg/ml de fungizone (tous en concentrations finale), contenant environ 100 000 cellules par ml, à +37°C en atmosphère contenant 5% de CO₂ pendant 16h. Lorsque les cellules arrivent à 75% de confluence, le milieu de culture est retiré. Les cellules COS sont ensuite infectées par du virus de la vaccine (souche WR) à une à une multiplicité d'infection (moi) de 3 DICC₅₀/cellule, puis les boîtes sont incubées pendant 1 h. Les cultures sont ensuite lavées avec du milieu DMEM sans sérum. On ajoute alors dans chaque boîte 400 µl de mélange plasmide/Lipofectine/Optimem (8 µl de Lipofectine, 192 µl d'Optimem et 200 µl d'eau distillée contenant 8 µg de plasmide), le plasmide étant pJCA166. Les boîtes sont incubées à +37°C en atmosphère contenant 5% de CO₂ 4 à 6h en les agitant toutes les 30 min. On ajoute ensuite dans chaque boîte du milieu DMEM supplémenté de 10% de sérum de veau foetal, et les boîtes sont mises à incuber à +37°C en atmosphère contenant 5% de CO₂ pendant 16h.

Les cellules peuvent alors être récoltées, congelées et stockées à -20°C.

La sélection des virus de la vaccine recombinés s'effectue sur des cultures de cellules humaines 143 TK- (accessibles auprès de l'American Type Culture Collection sous le numéro d'accès CRL-8303) dans des boîtes de 6 cm contenant 5 ml de milieu de culture DMEM, incubées à +37°C en atmosphère contenant 5% de CO₂ pendant 16h.

La suspension de transfection obtenue précédemment est ajoutée. Après une incubation d'1h à +37°C, 25 µg de 5-bromodésoxyuridine (BUdR) par ml est ajouté afin de sélectionner les virus de la vaccine recombinés TK-. L'incubation est prolongée pendant 48h afin de permettre le développement des cellules recombinées. 3 cycles successifs de sélection/purification de virus de la vaccine recombinés sont réalisés.

Des plaques de 24 puits sont ensemencées de cellules 143 TK- avec du milieu DMEM contenant 25 µg de BUdR par ml. Après 2h d'incubation à +37°C environ 10 plages reprises chacune dans 2 µl de tampon PBS sont transférées dans 10 puits. Les plaques sont alors incubées pendant 48-72h.

Après incubation, une réaction d'ACP est pratiquée sur le surnageant de culture de chaque puit en utilisant le protocole « Gene Releaser » (Cambio) avec les oligonucléotides JCA305 et JCA308.

Pour les puits trouvés positifs le virus recombiné subit un nouveau cycle de purification. Le virus recombiné désigné vV100 est amplifiée et stocké à -70°C ou à -20°C.

### Exemple 7 : Construction des virus recombinés de la vaccine pour les types O, C et A

Les constructions des virus recombinés de la vaccine sont obtenues pour les types O, C et A des virus aphteux comme cela est décrit dans l'exemple 6.

Pour le type O :
La réaction ACP est réalisée en utilisant le plasmide pJCA162 (exemple 4) comme matrice et les oligonucléotides suivants :
   JCA324 (37 mer) (SEQ ID NO 20) :
   5' TTTTGAATTCATGGGGGCTGGACAATCCAGTCCAGCG 3'
   et JCA325 (41 mer) (SEQ ID NO 21) :
   5' TTTTGAATTCATAAAAATCAAAGCTTGGTTTTGCGCATCAC 3'
   pour amplifier un fragment d'environ 3470 pb.

Après digestion et isolement, le fragment EcoRI-EcoRI de 3448 pb est inséré dans le plasmide pvFOHC, préalablement digéré par EcoRI, pour donner le plasmide donneur pJCA167.

La recombinaison est effectué selon la technique décrite à l'exemple 6. Des plages positives sont sélectionnées par réaction d'ACP avec les oligonucléotides JCA309 et JCA312. Une plage est amplifiée et le stock de virus recombiné obtenu est désigné vV101.

Pour le type C :
La réaction ACP est réalisée en utilisant le plasmide pJCA163 (exemple 4) comme matrice et les oligonucléotides suivants :
   JCA326 (37 mer) (SEQ ID NO 22) :
   5' TTTTGAATTCATGGGAGCTGGGCAATCCAGCCCAGCG 3'
   et JCA327 (41 mer) (SEQ ID NO 23) :
   5' TTTTGAATTCATAAAAATCAAAGCTTGGTTTTGCGCATTAC 3'
   pour amplifier un fragment d'environ 3460 pb.

Après digestion et isolement, le fragment EcoRI-EcoRI de 3439 pb est inséré dans le plasmide pvFOHC, préalablement digéré par EcoRI, pour donner le plasmide donneur pJCA168.

La recombinaison est effectué selon la technique décrite à l'exemple 6. Des plages positives sont sélectionnées par réaction d'ACP avec les oligonucléotides JCA313 et JCA316. Une plage est amplifiée et le stock de virus recombiné obtenu est désigné vV102.

Pour le type A :
La réaction ACP est réalisée en utilisant le plasmide pJCA164 (exemple 4) comme matrice et les oligonucléotides suivants :
   JCA328 (37 mer) (SEQ ID NO 24) :
   5' TTTTGAATTCATGGGGGCCGGGCAATCCAGTCCGGCG 3'
   et JCA329 (44 mer) (SEQ ID NO 25) :
   5' TTTTGAATTCATAAAAATCAGCGGCGGAACAGCGCTTTGTCCTC 3'
   pour amplifier un fragment d'environ 3550 pb.

Après digestion et isolement, le fragment EcoRI-EcoRI d'environ 3530 pb est inséré dans le plasmide pvFOHC, préalablement digéré par EcoRI, pour donner le plasmide donneur pJCA169.

La recombinaison est effectué selon la technique décrite à l'exemple 6. Des plages positives sont sélectionnées par réaction d'ACP avec les oligonucléotides JCA317 et JCA320. Une plage est amplifiée et le stock de virus recombiné obtenu est désigné vV103.

Variante pour le type A :
La réaction ACP est réalisée en utilisant le plasmide pJCA165 (exemple 5) comme matrice et les oligonucléotides suivants :
   JCA328 (SEQ ID NO 24) (37 mer)
   et JCA325 (SEQ ID NO 21) (37 mer)
   pour amplifier un fragment d'environ 3480 pb.

Après digestion et isolement, le fragment EcoRI-EcoRI d'environ 3463 pb est inséré dans le plasmide pvFOHC, préalablement digéré par EcoRI, pour donner le plasmide donneur pJCA170.

La recombinaison est effectué selon la technique décrite à l'exemple 6. Des plages positives sont sélectionnées par réaction d'ACP avec les oligonucléotides JCA317 et JCA312. Une plage est amplifiée et le stock de virus recombiné obtenu est désigné vV104.

### Exemple 8 :1 Construction de virus recombinés de la vaccine pour l'expression in vitro (A10)

Le plasmide pJCA161 (exemple 3) est digéré par les enzymes de restriction EcoRV et BgIII. Après électrophorèse en gel d'agarose, un fragment EcoRV-BgIII d'une taille d'environ 3450 pb est isolé. Ce fragment est rendu « bouts francs » par traitement avec la polymérase Klenow, puis ligaturé dans le plasmide pBG200 (Abrams et al., 1995, J. Gen. Virol., 76, 3089-3098) ce dernier étant préalablement digéré par BamHl et rendu bouts francs par traitement avec la polymérase Klenow, pour donner le plasmide donneur pJCA171.

Le plasmide pBG200 comporte le promoteur du bactériophage T7, le terminateur de transcription de T7 et deux bras du gène TK du virus de la vaccine, l'un en amont de ce promoteur et l'autre en aval du terminateur (l'homme de l'art peut s'inspirer de Fuerst et al., Molecular and Cellular Biology, 1987, 7, 2538-2544 pour la construction de pBG200). Le site d'insertion BamHI sur le plasmide pBG200 se trouve entre le promoteur et le terminateur de T7.

La recombinaison est effectué selon la technique décrite à l'exemple 6. Des plages positives sont sélectionnées par réaction d'ACP avec les oligonucléotides JCA305 et JCA308. Une plage est amplifiée et le stock de virus recombiné obtenu est désigné vV105.

La conservation du stock de virus recombiné se fait à -20°C ou - 70°C.

### Exemple 9 : Construction des virus recombinés de la vaccine pour l'expression in vitro des types O, C et A

Les constructions des virus recombinés de la vaccine pour l'expression *in vitro* sont obtenues pour les types O, C et A des virus aphteux comme cela est décrit dans l'exemple 8.

Pour le type O :
Le plasmide pJCA162 (exemple 4) est digéré par les enzymes de restriction EcoRV et BgIII. Après isolement, un fragment EcoRV-BgIII d'une taille d'environ 3450 pb est rendu « bouts francs » par traitement avec la polymérase Klenow, puis ligaturé dans le plasmide pBG200 préalablement digéré par BamHI et rendu bouts francs par traitement avec la polymérase Klenow, pour donner le plasmide donneur pJCA172.

Une plage sélectionnée par réaction d'ACP avec les oligonucléotides JCA309 et JCA312 est amplifiée et le stock de virus recombiné obtenu est désigné vV106.

Pour le type C :
Le plasmide pJCA163 (exemple 4) est digéré par les enzymes de restriction EcoRV et BgIII. Après isolement, un fragment EcoRV-BgIII d'une taille d'environ 3440 pb est rendu « bouts francs » par traitement avec la polymérase Klenow, puis ligaturé dans le plasmide pBG200 préalablement digéré par BamHI et rendu bouts francs par traitement avec la polymérase Klenow, pour donner le plasmide donneur pJCA173.

Une plage sélectionnée par réaction d'ACP avec les oligonucléotides JCA313 et JCA316 est amplifiée et le stock de virus recombiné obtenu est désigné vV107.

Pour le type A :
Le plasmide pJCA164 (exemple 4) est digéré par les enzymes de restriction EcoRV et BgIII. Après isolement, un fragment EcoRV-BgIII d'une taille d'environ 3520 pb est rendu « bouts francs » par traitement avec la polymérase Klenow, puis ligaturé dans le plasmide pBG200 préalablement digéré par BamHI et rendu bouts francs par traitement avec la polymérase Klenow, pour donner le plasmide donneur pJCA174.

Une plage sélectionnée par réaction d'ACP avec les oligonucléotides JCA317 et JCA320 est amplifiée et le stock de virus recombiné obtenu est désigné vV108.

Variante pour le type A :
Le plasmide pJCA165 (exemple 5) est digéré par les enzymes de restriction EcoRV et BgIII. Après isolement, un fragment EcoRV-BgIII d'une taille d'environ 3450 pb est rendu « bouts francs » par traitement avec la polymérase Klenow, puis ligaturé dans le plasmide pBG200 préalablement digéré par BamHI et rendu bouts francs par traitement avec la polymérase Klenow, pour donner le plasmide donneur pJCA175.

Une plage sélectionnée par réaction d'ACP avec les oligonucléotides JCA317 et JCA312 est amplifiée et le stock de virus recombiné obtenu est désigné vV109.

### Exemple 10 : Production et purification des capsides virales vides

Des cellules rénales de lapin RK13 (accessibles auprès de l'American Type Culture Collection sous le numéro d'accès CCL-37) sont mises en culture à 37°C dans 20 Falcons 175 cm² avec 20 ml de milieu DMEM supplémenté de 10 % de sérum de veau foetal, 2 mM du glutamine, 500 Ul/µg/ml de penicilline/streptomycine, 12,5 µg/ml de fungizone, chaque Falcon contient environ 2 10⁷ cellules à confluence.

Les virus recombinés de la vaccine vTF7-3 et vV108 (exemple 9) sont alors ajoutées chacun à une multiplicité d'infection (moi) de 10 DICC₅₀/cellule dans chaque Falcon. La culture virale est maintenue à 37°C pendant environ 24 heures jusqu'à l'obtention d'un effet cytopathogène à 100%.

Le virus recombiné de la vaccine vTF7-3 (accessible auprès de l'ATCC sous le numéro VR-2153) contient l'ARN polymérase du bactériophage T7 sous le contrôle du promoteur p7,5K du virus de la vaccine (Fuerst et al., 1986, Proc. Natl. Acad. Sci. USA, 83, 8122-8126).

La production de l'ARN polymérase T7 induit l'expression de l'insert sous contrôle du promoteur T7. Le précurseur P1 et la protéase 3C des virus aphteux sont ainsi produits et les capsides vides s'auto-assemblent.

La suspension virale est récoltée puis clarifiée par centrifugation (4 000 rotations par minute (rpm), pendant 30 min, à 4°C).

Le culot est resuspendu dans 30 ml de tampon phosphate (40 mM de phosphate de sodium, 100 mM de chlorure de sodium, pH7,6) à 0°C, contenant 0,5% de Nonidet P40 (Roche, Cat. No. 1 754 599). La lyse cellulaire est effectuée à 0°C sur glace pendant 20 min.

Les débris cellulaires sont récoltés après centrifugation à 10 000 rpm, pendant 20 min, à 4°C. Le surnageant est conservé à 0°C, sur glace. Les débris cellulaires, sont re-suspendus dans 6 ml de tampon phosphate. Une extraction au chloroforme (à volume égal) est effectuée. La phase aqueuse obtenue de cette extraction mélangée au surnageant obtenu précédemment est déposée sur un coussin de saccharose 15% (2 ml) et centrifugée avec un rotor Beeckman SW28 (28 000 rpm, 5 heures, 4°C). Le culot est repris dans 1 ml de tampon phosphate et stocké à 4°C.

Le culot obtenu est resuspendu, puis traité avec 20 µl de RNase (10 mg/ml) sur glace pendant 10 min. 10 µl de Nonidet P40 à 10% sont alors ajoutés et le tout est laissé 10 min sur la glace. Une extraction au chloroforme à volume égale est alors pratiquée sur la suspension. La phase aqueuse (1 ml) est récupérée et déposé sur un gradient de saccharose 15-45% (environ 12 ml) et centrifugé avec un rotor Beeckman SW40 (40 000 rpm, 5 heures, 12°C ou 18 000 rpm, 16 heures, 12°C).

Le gradient est ensuite fractionné en 14 fractions de 0,8 ml. On mesure l'absorbance à une longueur d'onde de 220 nm. Les fractions correspondant au pic d'absorbance sont récoltées. Ces fractions contiennent les capsides virales vides A24. La spécificité des protéines récoltées est vérifiée par Western Blot. Les capsides virales vides des sous-types O1K, A10, C Spain Olot et A24/A10 sont obtenues en procédant de manière identique à ce qui est décrit dans cet exemple, respectivement en remplaçant les virus recombinés de la vaccine vV108 par vV106 (exemple 9), vV105 (exemple 8), vV107 (exemple 9) et vV109 (exemple 9).

Les fractions protéiques ainsi obtenues sont conservées à 4°C avant leur utilisation dans des vaccins.

### Exemple 11 : Production de vaccins sous-unités

13,2 ml des fractions du gradient de saccharose contenant au total 165 µg de capsides vides de virus aphteux, obtenues à l'exemple 10, sont formulés avec 14,01 ml de DMEM avec 27,5 ml d'hydroxyde d'aluminium (Al(OH)₃) à 1,5% et avec 0,29 ml de saponine.

Les 55 ml ainsi obtenus sont ensuite répartis dans deux flacons, 30 ml dans le premier et 25 ml dans le deuxième. Une dose de 5 ml comprend 15 µg de capsides virales vides avec 360 unités hémolytiques de saponine.

La quantité de particules vides est déterminée par spectrophotométrie en mesurant l'adsorption à 220 nm en utilisant comme standard une solution d'albumine bovine (BSA).

### Exemple 12 : Production de vaccins recombinés

Pour la préparation de vaccins, les virus recombinés de la vaccine vV100 à vV105 (exemples 6 et 7) peuvent être adjuvés avec des solutions de carbomère. Le carbomère préféré est le Carbopol^{™} 974P fabriqué par BF Goodrich, Ohio, USA (poids moléculaire d'environ 3 000 000).

Une solution stock à 1,5 % de Carbopol^{™} 974P est initialement préparée dans de l'eau distillée contenant 1 g/l de chlorure de sodium. Cette solution stock est alors utilisée pour l'élaboration d'une solution à 4 mg/ml de Carbopol^{™} 974P en eau physiologique. La solution stock est mélangée au volume adéquat d'eau physiologique, soit en une étape unique soit en plusieurs étapes successives, la valeur du pH est ajustée à chaque étape avec une solution d'hydroxyde de sodium 1 N (ou encore plus concentrée) afin d'obtenir une valeur finale de pH de 7,3-7,4.

La solution de Carbopol^{™} 974P prête à l'emploi ainsi obtenue peut être utilisée pour reprendre des virus recombinés lyophilisés ou pour diluer des solutions stocks concentrées de virus recombinés. Par exemple, pour obtenir une suspension virale contenant 10⁸ pfu par dose de 1 ml, on dilue une solution virale stock de manière à obtenir un titre de 10^{8,3} pfu/ml, puis on dilue à parties égales avec ladite solution de Carbopol^{™} 974P prête à l'emploi à 4 mg/ml.

### Exemple 13 : Production de vaccins ADN

Une solution d'ADN contenant un ou plusieurs plasmides pJCA161 à pJCA165 (exemples 3 et 4) est concentrée par précipitation éthanolique comme décrit dans Sambrook et al (1989). Le culot d'ADN est repris par une solution de NaCl 0.9% de façon à obtenir une concentration de 1 mg/ml. Une solution de DMRIE-DOPE à 0,75mM est préparée par reprise d'un lyophilisat de DMRIE-DOPE par un volume adapté d'H₂O stérile (DMRIE ou N-(2-hydroxyéthyl)-N,N-diméthyl-2,3-bis(tetradécyloxy)-1-propanammonium (WO-A-9634109) ; DOPE ou dioléoyl-phosphatidyl-éthanolamine (Behr J. P., 1994, Bioconjugate Chemistry, 5, 382-389)).

La formation des complexes ADN plasmidique-lipide est réalisée par dilution à parties égales de la solution de DMRIE-DOPE 0.75 mM par la solution d'ADN à 1 mg/ml dans NaCl 0.9%. La solution d'ADN est introduire progressivement à l'aide d'une aiguille sertie 26G le long de la paroi du flacon contenant la solution de lipide cationique de façon à éviter la formation de mousse. On procède à une agitation douce dès que les deux solutions sont mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes avant de procéder à l'immunisation des animaux.

### Exemple 14 : Contrôle sur bovins

Le vaccin contre le virus aphteux A24, obtenu à l'exemple 11 à partir des capsides virales vides exprimées par vV109, est administré à un groupe de 6 bovins.

L'injection est réalisée par voie sous-cutanée, de chaque côté du cou, face aux épaules. Les 5 premiers animaux reçoivent une dose de 5 ml (2 × 2,5 ml), le 6^{ème} animal reçoit 4 ml (2 × 2,0 ml). Ce 6^{ème} animal est tatoué à l'oreille « UC10 ». Une deuxième injection est administrée par voie sous-cutanée de chaque côté du cou à chaque animal 31 jours après la primo-vaccination (4 ml pour les 5 premiers animaux (2 × 2,0 ml) et 0,5 ml (2 × 0,25 ml) pour le 6^{éme} animal).

Des prélèvements sanguins sont pratiqués sur les animaux vaccinés à J0 (jour de la primo-vaccination), J6, J13, J20, J31, J38, J42, J52 et immédiatement avant leur sacrifice.

Tous les animaux vaccinés et 2 animaux contrôles non-vaccinés sont éprouvés par voie intradermolinguale (10 × 0,10 ml par langue) avec des virus aphteux A24 à un titre de 10^{4,4} doses infectieuses/ml (titre sur cellules thyroïdiennes de bovin). L'épreuve a lieu 42 jours après la primo-vaccination.

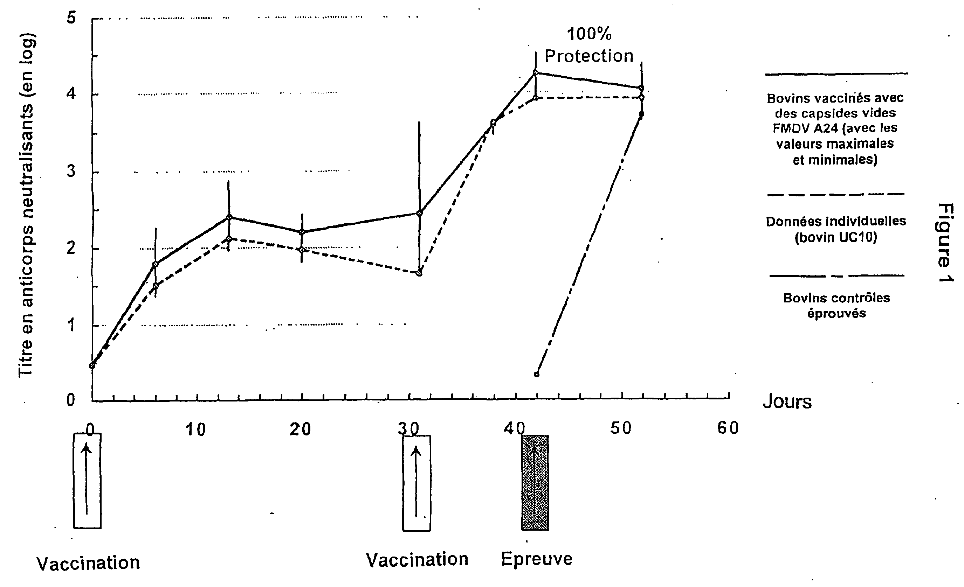

Un suivi des températures (tableau 2) et des signes de fièvre aphteuse (tableau 1) au niveau de la langue, de la bouche et des pieds est assuré quotidiennement pour chaque animal. Les titres en anticorps neutralisants anti-virus aphteux A24 sont suivis (Figure 1).

**Tableau 1 : Suivi des signes cliniques de fièvre aphteuse sur les bovins après épreuve virulente**

| Animal /jour après épreuve | 0 | 1 | 2 | 3 | 4 | 5 | 6 | 7 | 8 | 9 | 10 |
|---|---|---|---|---|---|---|---|---|---|---|---|
| Vacciné UC6 | T0 4F0 | T1 4F0 | T1 4F0 | T1 4F0 | T1 4F0 | T1 4F0 | ND | T1 4F0 | T0 4F0 | ND | T0 4F0 |
| Vacciné UC7 | T0 4F0 | T1 4F0 | T1 4F0 | T1 4F0 | T1 4F0 | T1 4F0 | ND | T1 4F0 | T0 4F0 | ND | T0 4FO |
| Vacciné UC8 | T0 4F0 | T1 4F0 | T1 4F0 | T1 4F0 | T1 4F0 | T1 4F0 | ND | T1 4F0 | T0 4F0 | ND | T0 4F0 |
| Vacciné UC9 | T0 4F0 | T1 4F0 | T1 4F0 | T1 4F0 | T1 4F0 | T1 4F0 | ND | T1 4F0 | T0 4F0 | ND | T0 4F0 |
| Vacciné UC10 | T0 4F0 | T1 4F0. | T1 4F0 | T1 4F0 | T1 4F0 | T1 4F0 | ND | T1 4F0 | T0 4F0 | ND | T0 4F0 |
| Vacciné UC11 | T0 4F0 | T1 4F0 | T1 4F0 | T1 4F0 | T1 4F0 | T1 4F0 | ND | T1 4F0 | T0 4F0 | ND | T0 4F0 |
| Contrôle UC79 | T0 4F0 | T1 4F0 | T2 4F0 | T2 4F0 | T2 4F0 | T2 4F+ | T2 4F+ | T2 4F+ | T2 4F+ | T2 4F+ | T2 4F+ |
| Contrôle UC80 | T0 4F0 | T1 4F0 | T2 4F0 | T2 4F0 | T2 4F0 | T2 4F+ | T2 4F+ | T2 4F+ | T2 4F+ | T2 4F+ | T2 4F+ |

| | | | | | | | | | | | |
|---|---|---|---|---|---|---|---|---|---|---|---|
| Significations des codes : T0: langue saine, aucun signe de réplication virale, aucune trace aux points d'injection T1 : langue saine, aucun signe de réplication virale, uniquement des trauma aux points d'injection T2 : présence de lésions primaires sur la langue T3 : présence de lésions primaires et secondaires sur la langue ou sur d'autres régions de la bouche 4F0 : aucun signe de fièvre aphteuse au niveau des 4 pieds 4F+ : présence de vésicules sur les 4 pieds. ND : pas d'examen clinique pratiqué | | | | | | | | | | | |

Ces résultats montrent que les animaux vaccinés sont tous protégés contre une infection par le virus aphteux A24, même localement au niveau des points d'injection. Le bovin UC10 qui a reçu une injection de rappel plus faible que les autres est également protégé. A l'inverse, les animaux contrôles sensibles à l'infection par le virus, développent la maladie de façon visible dans la bouche dès le deuxième jour après épreuve et sur les pieds dès le cinquième jour après épreuve.

**Tableau 2 : suivi des températures (en °C) des bovins après épreuve virulente**

| Animal / jour après épreuve | 0 | 1 | 2 | 3 | 4 | 5 | 6 | 7 | 8 | 9 | 10 |
|---|---|---|---|---|---|---|---|---|---|---|---|
| Vacciné UC6 | 38,6 | 38,6 | 38,2 | 37,9 | 38,2 | 38,6 | 38,5 | 38,7 | 38,5 | 39,1 | 39,1 |
| Vacciné UC7 | 38,7 | 38,5 | 38,4 | 38,2 | 38,3 | 38,2 | 38,3 | 38,5 | 38,6 | 38,4 | 38,0 |
| Vacciné UC8 | 38,3 | 39,0 | 38,3 | 38,5 | 38,6 | 38,6 | 38,5 | 38,3 | 38,2 | 38,0 | 38,6 |
| Vacciné UC9 | 38,8 | 38,7 | 39,0 | 38,6 | 38,4 | 38,5 | 38,3 | 38,4 | 38,6 | 38,8 | 39,0 |
| Vacciné UC10 | 39,3 | 38,8 | 38,9 | 38,6 | 38,6 | 38,7 | 38,8 | 39,0 | 38,7 | 39,2 | 39,1 |
| Vacciné UC11 | 38,6 | 38,8 | 38,4 | 38,3 | 38,3 | 38,0 | 38,2 | 38,0 | 38,3 | 38,7 | 38,8 |
| Contrôle UC79 | 39,0 | 40,7 | 41,0 | 39,5 | 39,6 | 38,8 | 38,4 | 38,6 | 38,4 | 38,6 | 38,8 |
| Contrôle UC80 | 39,1 | 39,8 | 40,6 | 39,9 | 39,4 | 38,9 | 38,6 | 38,5 | 38,9 | 38,8 | 38,6 |

Ces résultats montrent que les bovins vaccinés ne présentent pas d'hyperthermie, même le bovin UC10 qui a reçu une injection de rappel plus faible que les autres. A l'inverse, les bovins contrôles présentent une hyperthermie, dont le maximum apparaît deux jours après l'épreuve, puis un retour à des températures corporelles normales.

La Figure 1 montre que les bovins vaccinés développent une forte réponse en anticorps neutralisants anti-virus aphteux A24 avec un premier pic à environ 13 jours après la primo-vaccination. Après l'injection de rappel, une forte réponse en anticorps neutralisants est observée. Ceci est également observé chez le bovin UC10 qui a reçu une injection de rappel plus faible que les autres. Après épreuve le titre en anticorps n'augmente pas ce qui indique que le virus FDMV A24 ne se réplique pas suffisamment pour stimuler la réponse en anticorps.

En conclusion, le vaccin contre la fièvre aphteuse A24 réalisé à partir de capsides virales vides obtenues de vecteurs d'expression recombinés virus de la vaccine induit une réponse immunitaire primaire et secondaire chez les bovins. Après épreuve, ces bovins sont totalement protégés contre la fièvre aphteuse et contre une réplication virale.

### Exemple 15 :Mutagenèse sur la construction A10

Une mutagenèse dirigée sur la construction A10 est effectuée au moyen d'oligonucléotides et de réactions d'ACP dans le but de remplacer le codon codant pour l'histidine 179 (position dans la polyprotéine codée par le plasmide pJCA161, exemple 3 ; la méthionine initiatrice étant numérotée 1) par un codon codant pour une cystéine.

Une réaction ACP est réalisée en utilisant le plasmide pJCA161 comme matrice et les oligonucléotides suivants :
JCA309 (37 mer) (SEQ ID NO 5)
et JCA330 (27 mer) (SEQ ID NO 26) :
5' TGAGTCCACCAGGCACCCGAAGACACC 3'
pour amplifier un fragment de 559 pb. Ce fragment est appelé fragment L.

Les conditions du premier cycle de la réaction d'ACP sont 95°C pendant 2 min, puis 62°C pendant 2 min et 72°C pendant 3 min.

Les conditions des 35 cycles suivants de la réaction d'ACP sont les mêmes que celles décrites dans l'exemple 6.

Une deuxième réaction ACP est réalisée en utilisant le plasmide pJCA161 comme matrice et les oligonucléotides suivants :
JCA331 (27 mer) (SEQ ID NO 27) :
5' GGTGTCTTCGGGTGCCTGGTGGACTCA 3'
et JCA332 (36 mer) (SEQ ID NO 28) :
5' AAAGTCTTTGCCGGCGCTAGCCGACACTAACAAGGT 3'
pour amplifier un fragment de 1020 pb. Ce fragment est appelé fragment M.

Les conditions de la réaction d'ACP sont les mêmes que celles décrites dans l'exemple 6.

Une troisième réaction ACP est réalisée en utilisant les fragments L et M comme matrice, et les oligonucléotides JCA309 (SEQ ID NO 5) et JCA332 (SEQ ID NO 28) pour amplifier un fragment de 1552 pb. Les conditions de la réaction d'ACP sont les mêmes que celles décrites dans l'exemple 6.

Ce fragment est ensuite digéré à l'aide de EcoRV et de Nhel, pour isoler, après électrophorèse en gel d'agarose, le fragment. EcoRV-Nhel de 1527 pb. Ce fragment est appelé fragment N.

Le plasmide pJCA161 est digéré par Nhel puis par EcoRV pour isoler, après électrophorèse en gel d'agarose, le fragment Nhel-EcoRV d'environ 6800 pb. Ce fragment et le fragment N sont ligaturés ensemble pour donner le plasmide pJCA176 (8327 pb). Ce plasmide contient un insert codant pour la partie de la polyprotéine A10 suffisante pour générer des protéines de capside mutées thermostables capables de s'auto-assembler.

Le plasmide pJCA176 est utilisé pour la construction d'un virus recombiné vaccine selon l'exemple 8, le fragment EcoRV-BgIII étant obtenu dans ce cas à partir du plasmide pJCA176 et non pas à partir du plasmide pJCA161 Le virus recombiné ainsi obtenu est appelé vV110.

### Exemple 16 : Mutagenèse sur la construction 01 K

Une mutagenèse dirigée sur la construction 01 K est effectuée au moyen d'oligonucléotides et de réactions d'ACP comme cela est décrit dans l'exemple 15, dans le but de remplacer le codon codant pour la sérine 179 (position dans la polyprotéine codée par le plasmide pJCA162, exemple 4; la méthionine initiatrice étant numérotée 1) par un codon codant pour une cystéine.

Une réaction d'ACP est réalisée en utilisant le plasmide pJCA162 comme matrice et les oligonucléotides suivants :
JCA305 (37 mer) (SEQ ID NO 1)
et JCA333 (27 mer) (SEQ ID NO 29):
5' CGAGTCAGTCAGGCAGCCGTAGACACC 3'
pour amplifier un fragment de 559 pb. Ce fragment est appelé fragment O.

Une deuxième réaction d'ACP est réalisée en utilisant le plasmide pJCA162 comme matrice et les oligonucléotides suivants :
JCA334 (27 mer) (SEQ ID NO 30) :
5' GGTGTCTACGGCTGCCTGACTGACTCG 3'
et JCA335 (36 mer) (SEQ ID NO 31):
5' AGACGTCCGTGTGTTGGCGCCTCTGGATCTGTGTTT 3'
pour amplifier un fragment de 1147 pb. Ce fragment est appelé fragment P. Une troisième réaction ACP est réalisée en utilisant les fragments 0 et P comme matrice, et les oligonucléotides JCA305 (SEQ ID NO 1) et JCA335 (SEQ ID NO 31) pour amplifier un fragment de 1679 pb.

Ce fragment est ensuite digéré à l'aide de EcoRV et de NarI, pour isoler, après électrophorèse en gel d'agarose, le fragment EcoRV-Narl de 1654 pb. Ce fragment est appelé fragment Q.

Le plasmide pJCA162 est digéré par Narl puis par EcoRV pour isoler, après électrophorèse en gel d'agarose, le fragment Narl-EcoRV d'environ 6670 pb. Ce fragment et le fragment Q sont ligaturés ensemble pour donner le plasmide pJCA177 (8327 pb). Ce plasmide contient un insert codant pour la partie de la polyprotéine O1K suffisante pour générer des protéines de capside mutées thermostables capables de s'auto-assembler.

Le plasmide pJCA177 est utilisé pour la construction d'un virus recombiné vaccine selon l'exemple 9, le fragment EcoRV-BgIII étant obtenu dans ce cas à partir du plasmide pJCA177 et non pas à partir du plasmide pJCA162 Le virus recombiné ainsi obtenu est appelé vV111.

### Exemple 17 : Mutagenèse sur la construction C Spain Olot

Une mutagenèse dirigée sur la construction C Spain Olot est effectuée au moyen d'oligonucléotides et de réactions d'ACP comme cela est décrit dans l'exemple 15, dans le but de remplacer le codon codant pour la glycine 179 (position dans la polyprotéine codée par le plasmide pJCA163, exemple 4 ; la méthionine initiatrice étant numérotée 1) par un codon codant pour une cystéine.

Une réaction ACP est réalisée en utilisant le plasmide pJCA163 comme matrice et les oligonucléotides suivants :
JCA313 (37 mer) (SEQ ID NO 9)
et JCA336 (27 mer) (SEQ ID NO 32) :
5' TGACTTGACGAGGCACCCGTAAACACC 3'
pour amplifier un fragment de 559 pb. Ce fragment est appelé fragment R.

Une deuxième réaction ACP est réalisée en utilisant le plasmide pJCA163 comme matrice et les oligonucléotides suivants :
JCA337 (27 mer) (SEQ ID NO 33) :
5' GGTGTTTACGGGTGCCTCGTCAAGTCA 3'
et JCA338 (36 mer) (SEQ ID NO 34) :
5' GTAGTACTGGGCCAAGCCGGCCAAGTAGGTGTTTGA 3'
pour amplifier un fragment de 681 pb. Ce fragment est appelé fragment S.

Une troisième réaction ACP est réalisée en utilisant les fragments R et S comme matrice, et les oligonucléotides JCA313 (SEQ ID NO 9) et JCA338 (SEQ ID NO 34) pour amplifier un fragment de 1213 pb.

Ce fragment est ensuite digéré par EcoRV et Nael, pour isoler, après électrophorèse en gel d'agarose, le fragment EcoRV-Nael d'environ 1190 pb. Ce fragment est appelé fragment T.

Le plasmide pJCA163 est digéré par Nael puis par EcoRV pour isoler, après électrophorèse en gel d'agarose, le fragment Nael-EcoRV d'environ 7120 pb. Ce fragment et le fragment T sont ligaturés ensemble pour donner le plasmide pJCA178 (8312 pb). Le plasmide pJCA178 contient un insert codant pour la partie de la polyprotéine C Spain Olot suffisante pour générer des protéines de capside mutées thermostables capables de s'auto-assembler.

Le plasmide pJCA178 est utilisé pour la construction d'un virus recombiné vaccine selon l'exemple 9, le fragment EcoRV-BgIII étant obtenu dans ce cas à partir du plasmide pJCA178 et non pas à partir du plasmide pJCA163 Le virus recombiné ainsi obtenu est appelé vV112.

### Exemple 18 : Mutagenèse sur la construction A24

Une mutagenèse dirigée sur la construction A24 est effectuée au moyen d'oligonucléotides et de réactions d'ACP comme cela est décrit dans l'exemple 15, dans le but de remplacer le codon codant pour l'histidine 179 (position dans la polyprotéine codée par le plasmide pJCA164, exemple 4 ; la méthionine initiatrice étant numérotée 1) par un codon codant pour une cystéine.

Une réaction ACP est réalisée en utilisant le plasmide pJCA164 comme matrice et les oligonucléotides suivants :
JCA317 (37 mer) (SEQ ID NO 13)
et JCA339 (27 mer) (SEQ ID NO 35) :
5' CGAGTCCACCAAGCATCCAAAGACACC 3'
pour amplifier un fragment de 559 pb. Ce fragment est appelé fragment U.

Une deuxième réaction ACP est réalisée en utilisant le plasmide pJCA164 comme matrice et les oligonucléotides suivants :
JCA340 (27 mer) (SEQ ID NO 36) :
5' GGTGTCTTTGGATGCTTGGTGGACTCG 3'
et JCA341 (36 mer) (SEQ ID NO 37) :
5' CCCAGGGTAGTTAGTCCTAGGCGGGTTGTACACCTT 3'
pour amplifier un fragment de 507 pb. Ce fragment est appelé fragment V. Une troisième réaction ACP est réalisée en utilisant les fragments U et V comme matrice, et les oligonucléotides JCA317 (SEQ ID NO 13) et JCA341 (SEQ ID NO 37) pour amplifier un fragment de 1039 pb.

Ce fragment est ensuite digéré par EcoRV et BlnI, pour isoler, après électrophorèse en gel d'agarose, le fragment EcoRV-BlnI d'environ 1014 pb. Ce fragment est appelé fragment W.

Le plasmide pJCA164 est digéré par BlnI puis par EcoRV pour isoler, après électrophorèse en gel d'agarose, le fragment BlnI-EcoRV d'environ 7360 pb. Ce fragment et le fragment W sont ligaturés ensemble pour donner le plasmide pJCA179 (d'une taille d'environ 8400 pb). Ce plasmide contient un insert codant pour la partie de la polyprotéine A24 suffisante pour générer des protéines de capside mutées thermostables capables de s'auto-assembler.

Le plasmide pJCA179 est utilisé pour la construction d'un virus recombiné vaccine selon l'exemple 9, le fragment EcoRV-BgIII étant obtenu dans ce cas à partir du plasmide pJCA179 et non pas à partir du plasmide pJCA164 Le virus recombiné ainsi obtenu est appelé vV113.

### Exemple 19 : Production et purification des capsides virales vides thermostables

Les capsides virales vides modifiées sous-types A10, O1K, C Spain Olot et A24 sont obtenues en procédant de manière identique à ce qui est décrit dans l'exemple 10, en remplaçant les virus recombinés de la vaccine vV108 par respectivement vV110 (exemple 15), vV111 (exemple 16), vV112 (exemple 17) et vV113 (exemple 18).

### Exemple 20 : Contrôle de thermostabilité

10 tubes contenant des capsides virales vides modifiées (exemple 19) de virus aphteux A10 sont préparés et quantifiés. Chaque tube contient 0,8 µg de capsides dans 0,5 ml de tampon phosphate à pH7,6.

Ces 10 tubes sont placés dans un bain-marie à 50°C pendant 1 heure. Ils sont ensuite refroidis. Chaque échantillon de capsides est déposé sur un gradient de saccharose (15-35%) et centrifugé pendant 2,5 heures à 12°C (40 000 rpm avec un rotor Beeckman SW40). Chaque gradient obtenu est alors fractionné en 12 fractions de 1 ml.

0,5 ml de chaque fraction est précipité en présence d'1 ml d'éthanol absolu à -20°C pendant 16 heures.

Le culot est collecté, séché, resuspendu dans un tampon de chargement (Maniatis et al., 1982, in: « Molecular cloning : a laboratory manual », Cold Spring Harbor Laboratory, Cold Spring Harbor, NY, USA) et déposé sur un gel d'électrophorèse SDS-PAGE 10% d'acrylamide. Les bandes des protéines après transfert sont révélées par un anticorps polyclonal de cobaye anti-140S A10 réagissant essentiellement avec la protéine VP1.

Les capsides vides migrent dans le gradient au niveau des fractions 7 et 8 alors que les capsides vides dégradées restent près du sommet du gradient au niveau de la fraction 11.

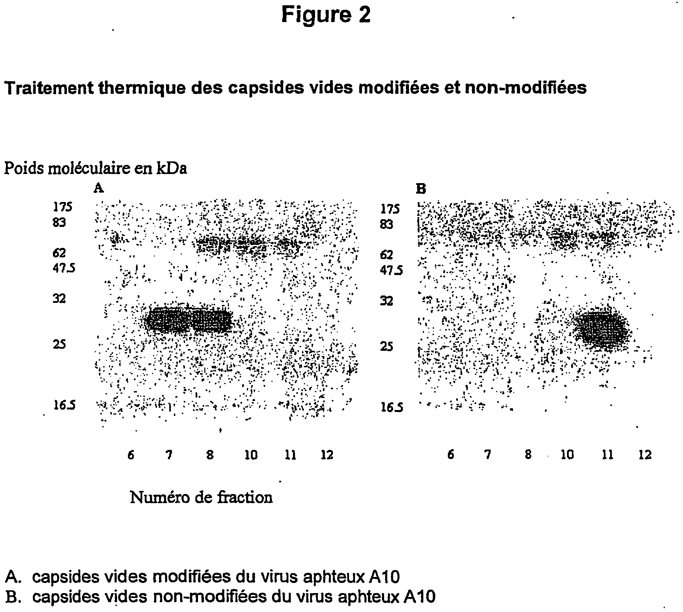

Le Western (Figure 2) montre que les capsides vides correspondant au mutant sont toujours assemblées après 1h à 50°C (gel A) alors que les capsides vides non mutées sont dégradées, n'ayant pas résistées au traitement thermique (gel B) comme on peut s'y attendre.

### Exemple 21 : Production de vaccins sous-unités contenant des capsides vides thermostables de virus aphteux

Les vaccins contenant des capsides virales vides modifiées des sous-types O1K, A10, C Spain Olot et A24 de virus aphteux sont obtenues en procédant de manière semblable à ce qui est décrit dans l'exemple 11, en remplaçant les capsides virales vides non-modifiées par les capsides virales vides modifiées (exemple 19).

## Revendications

1. Vaccin contre la fièvre aphteuse, comprenant une quantité efficace d'antigène consistant en des capsides vides du virus aphteux, obtenues *in vitro* par l'expression dans des cellules eucaryotes, de l'ADNc de la région P1 du génome du virus aphteux codant pour la capside et de l'ADNc de la région du génome du virus aphteux codant pour la protéase 3C, le vaccin comprenant en outre un véhicule ou excipient pharmaceutiquement acceptable sur le plan vétérinaire, dans lequel l'ADNc de la région P1 du génome du virus aphteux est modifié par remplacement du codon codant pour l'acide aminé 179 de la séquence d'acides aminés SEQ ID NO: 38, ou du codon codant pour l'acide aminé correspondant à l'acide aminé 179 de la séquence d'acides aminés SEQ ID NO: 38 par un codon codant pour une cystéine.

2. Vaccin selon la revendication 1, dans lequel l'ADNc de la région P1 du génome du virus aphteux est modifié par remplacement du codon codant pour l'acide aminé X₆ de la séquence X₁ Gly X₃ X₄ Gly X₆ Leu X₈ X₉ Ser X₁₁ X₁₂ Tyr Met dans laquelle
- X₄ et X₁₁ sont Tyr, His ou Phe,
- X₃, X₈ et X₁₂ sont Val, Met, Ile, Thr ou Ala,
- X₆ est His, Gln, Arg, Lys, Ser ou Gly,
- X₁ est His ou Lys
- X₉ est Asp, Glu ou Lys ;
par un codon codant pour une cystéine.

3. Vaccin selon la revendication 1 ou 2, dans lequel les capsides vides du virus aphteux comprennent les protéines VP0, VP1 et VP3.

4. Vaccin selon l'une quelconque des revendications 1 à 3, dans lequel l'ADNc codant pour P1 code aussi pour tout ou partie de 2A.

5. Vaccin selon la revendication 4, dans lequel l'ADNc code aussi pour une partie de 2B.

6. Vaccin selon l'une quelconque des revendications 1 à 5, dans lequel l'ADNc codant pour 3C code aussi pour tout ou partie des protéines 3B, pour une partie non fonctionnelle de 3D, ou à la fois pour tout ou partie des protéines 3B et pour une partie non fonctionnelle de 3D.

7. Vaccin selon l'une quelconque des revendications 1 à 6, dans lequel les capsides vides sont sous forme de sous-unités obtenues par expression de l'ADNc en cellules eucaryotes, par un vecteur d'expression *in vitro,* sous la dépendance d'un promoteur inductible ou d'un promoteur tardif d'origine virale.

8. Vaccin selon la revendication 7, dans lequel le promoteur est le promoteur du bactériophage T7 et les capsides vides sous forme de sous-unités sont obtenues par expression de l'ADNc en présence de l'expression de polymérase T7.

9. Vaccin selon l'une quelconque des revendications précédentes, dans lequel le vecteur d'expression est le virus de la vaccine.

10. Vaccin selon l'une quelconque des revendications précédentes, dans lequel le vaccin est formulé dans un milieu permettant la conservation des capsides vides du type DMEM.

11. Vaccin selon l'une quelconque des revendications 1 à 10, comprenant en outre un adjuvant.

12. Vaccin selon la revendication 11, dans lequel ledit vaccin comprend à titre d'adjuvant de l'hydroxyle d'alumine, de la saponine, de l'avridine, du DDA, un polymère de l'acide acrylique ou méthacrylique, un polymère d'anhydride maléique et de dérivé alcényle, ou du GM-CSF, ou dans lequel ledit vaccin est formulé sous forme d'une émulsion eau-dans-huile, huile-dans-eau ou eau-dans-huile-dans-eau.

13. Vaccin selon la revendication 12, dans lequel l'adjuvant comprend un carbomère.

14. Vaccin contre la fièvre aphteuse, comprenant un vecteur d'expression contenant de l'ADNc de la région P1 du génome du virus aphteux codant pour la capside et de l'ADNc de la région du génome du virus aphteux codant pour la protéase 3C, de façon à produire *in vivo* une quantité efficace d'antigène consistant en des capsides vides du virus aphteux, le vaccin comprenant en outre un véhicule ou excipient pharmaceutiquement acceptable sur le plan vétérinaire, dans lequel l'ADNc de la région P1 du génome du virus aphteux est modifié par remplacement du codon codant pour l'acide aminé 179 de la séquence d'acides aminés SEQ ID NO: 38, ou du codon codant pour l'acide aminé correspondant à l'acide aminé 179 de la séquence d'acides aminés SEQ NO: 38 par un codon codant pour une cystéine.

15. Vaccin selon la revendication 14, dans lequel l'ADNc de la région P1 du génome du virus aphteux est modifié par remplacement du codon codant pour l'acide aminé X₆ de la séquence X₁ Gly X₃ X₄ Gly X₆ Leu X₈ X₉ Ser X₁₁ X₁₂ Tyr Met dans laquelle
- X₄ et X₁₁ sont Tyr, His ou Phe,
- X₃, X₈ et X₁₂ sont Val, Met, Ile, Thr ou Ala,
- X₆ est His, Gln, Arg, Lys, Ser ou Gly,
- X₁ est His ou Lys
- X₉ est Asp, Glu ou Lys ;
par un codon codant pour une cystéine.

16. Vaccin selon la revendication 14 ou 15, dans lequel les capsides vides du virus aphteux comprennent les protéines VP0, VP1 et VP3.

17. Vaccin selon l'une quelconque des revendications 14 à 16, dans lequel l'ADNc codant pour P1 code aussi pour tout ou partie de 2A.

18. Vaccin selon la revendication 17, dans lequel l'ADNc code aussi pour une partie de 2B.

19. Vaccin selon l'une quelconque des revendications 14 à 18, dans lequel d'ADNc codant pour 3C code aussi pour tout ou partie des protéines 3B, pour une partie non fonctionnelle de 3D, ou à la fois pour tout ou partie des protéines 3B et pour une partie non fonctionnelle de 3D.

20. Vaccin selon la revendication 19, dans lequel le vecteur d'expression est un vecteur viral choisi parmi les poxvirus ou les adénovirus réplicatifs.

21. Vaccin selon la revendication 20, dans lequel le vecteur d'expression est un poxvirus et l'ADNc est placé sous la dépendance d'un promoteur tardif d'origine virale choisi parmi P11K du virus de la vaccine, P28K du virus de la vaccine, et P160K ATI du virus cowpox.

22. Vaccin selon l'une quelconque des revendications 14 à 19, dans lequel le vecteur d'expression est un vecteur plasmidique et l'ADNc est placé sous la dépendance d'un promoteur choisi dans le groupe consistant en le promoteur précoce du virus SV40, le promoteur LTR du virus de Sarcome de Rous, et un promoteur d'un gène du cytosquelette.

23. Vaccin selon l'une quelconque des revendications 14 à 22, comprenant en outre un adjuvant.

24. Vaccin selon la revendication 23, dans lequel ledit vaccin comprend un vecteur plasmidique et l'adjuvant comprend un lipide cationique contenant un sel d'ammonium quaternaire de formule
R₁OCH₂CH(OR₁)CH₂N⁺(CH₃)₂(R₂)X
dans laquelle R₁ est un radical aliphatique linéaire, saturé ou insaturé, ayant de 12 à 18 atomes de carbone, R₂ est un autre radical aliphatique contenant 2 ou 3 atomes de carbone, et X est un groupe hydroxyle ou amine.

25. Vaccin selon la revendication 24, dans lequel l'adjuvant comprend du DMRIE.

26. Vaccin selon la revendication 25, dans lequel le DMRIE est associé au DOPE pour former DMRIE-DOPE.

27. Vaccin selon la revendication 23, dans lequel ledit vaccin comprend un vecteur viral et l'adjuvant comprend un polymère de l'acide acrylique ou méthacrylique ou un polymère d'anhydride maléique et de dérivé alcényle.

28. Vaccin selon la revendication 23, dans lequel l'adjuvant contient GM-CSF.

29. Vaccin selon la revendication 23, dans lequel l'adjuvant contient un vecteur qui exprime *GM-CSF in vivo.*

30. Vaccin selon l'une quelconque des revendications 1 à 29, dans lequel ledit vaccin est destiné à être administré à un bovin

31. Vaccin selon l'une quelconque des revendications 1 à 30, pour son utilisation dans une méthode de vaccination d'un bovin.

32. Utilisation de capsides vides du virus aphteux, obtenues *in vitro* par l'expression dans des cellules eucaryotes, de l'ADNc de la région P1 du génome du virus aphteux codant pour la capside et de l'ADNc de la région du génome du virus aphteux codant pour la protéase 3C, pour la fabrication d'un vaccin contre la fièvre aphteuse destiné à être administré à un bovin, dans lequel l'ADNc de la région P1 du génome du virus aphteux est modifié par remplacement du codon codant pour l'acide aminé 179 de la séquence d'acides aminés SEQ ID NO: 38, ou du codon codant pour l'acide aminé correspondant à l'acide aminé 179 de la séquence d'acides aminés SEQ ID NO: 38 par un codon codant pour une cystéine.

## Claims

1. Vaccine against foot-and-mouth disease, comprising an effective amount of antigen consisting of empty capsids of foot-and-mouth disease virus obtained by expression *in vitro,* in eukaryotic cells, of the cDNA of the P1 region of the foot-and-mouth virus genome encoding the capsid and of the cDNA of the region of the foot-and-mouth virus genome encoding the 3C protease, said vaccine further comprising a veterinarily acceptable carrier or excipient, wherein the cDNA of the P1 region of the foot-and-mouth disease virus genome is modified by replacement of the codon coding for amino acid 179 of the amino acid sequence as set forth in SEQ NO:38 or of the codon coding for the amino acid corresponding to amino acid 179 of the amino acid sequence as set forth in SEQ NO:38 by a codon coding for a cysteine.

2. Vaccine according to claim 1, wherein the cDNA of the P1 region of the foot-and-mouth disease virus genome is modified by replacement of the amino acid X₆ of the sequence X₁ Gly X₃ X₄ Gly X₆ Leu X₈ X₉ Ser X₁₁ X₁₂ Tyr Met where
- X₄ and X₁₁ are Tyr, His or Phe,
- X₃, X₈ and X₁₂ are Val, Met, Ile, Thr or Ala,
- X₆ is His, Gln, Arg, Lys, Ser or Gly,
- X₁ is His or Lys,
- X₉ is Asp, Glu or Lys ;
by a codon coding for a cysteine.

3. Vaccine according to claim 1 or 2, wherein the empty capsids of foot-and-mouth disease virus comprise VP0, VP1, and VP3 proteins.

4. Vaccine according to any one of claims 1-3, wherein the cDNA encoding P1 also codes for all or part of 2A.

5. Vaccine according to claim 4, wherein the cDNA also codes for part of 2B.

6. Vaccine according to any one of claims 1-5, wherein the cDNA encoding 3C also codes for all or part of proteins 3B, for a non-functional part of 3D, or for both all or part of proteins 3B and a non-functional part of 3D.

7. Vaccine according to any one of claims 1-6, wherein the empty capsids are in the form of sub-units obtained by expression of the cDNA in eukaryotic cells, with an expression vector *in vitro*, under the control of an inducible promoter or a late promoter of viral origin.

8. Vaccine according to claim 7, wherein the promoter is T7 bacteriophage promoter, and wherein the empty capsids in the form of sub-units are obtained by expression of the cDNA in the presence of T7 polymerase.

9. Vaccine according to any of the preceding claims, wherein the expression vector is the vaccinia virus.

10. Vaccine according to any of the preceding claims, wherein said vaccine is formulated in a medium allowing conservation of the empty capsids of the DMEM type.

11. Vaccine according to any one of claims 1-10, further comprising an adjuvant.

12. Vaccine according to claim 11, wherein said vaccine comprises as an adjuvant aluminum hydroxide, saponine, avridine, DDA, a polymer of acrylic or methacrylic acid, a polymer of maleic anhydride and an alkenyl derivative, or GM-CSF, or wherein said vaccine is formulated in the form of a water-in-oil, oil-in-water or water-in-oil-in-water emulsion.

13. Vaccine according to claim 12, wherein said adjuvant comprises a carbomer.

14. Vaccine against foot-and-mouth disease, comprising an expression vector containing cDNA of the P1 region of the foot-and-mouth disease virus genome encoding the capsid and cDNA of the region of the the foot-and-mouth disease virus genome encoding the 3C protease, for the *in vivo* production of an effective amount of antigen consisting of empty capsids of the foot-and-mouth disease virus, said vaccine further comprising a veterinarily acceptable carrier or excipient, wherein the cDNA of the P1 region of the foot-and-mouth disease virus genome is modified by replacement of the codon coding for amino acid 179 of the amino acid sequence as set forth in SEQ ID NO:38 or of the codon coding for the amino acid corresponding to amino acid 179 of the amino acid sequence as set forth in SEQ ID NO:38 by a codon coding for a cysteine.

15. Vaccine according to claim 14, wherein the cDNA of the P1 region of the foot-and-mouth disease virus genome is modified by replacement of the amino acid X₆ of the sequence X₁ Gly X₃ X₄ Gly X₆ Leu X₈ X₉ Ser X₁₁ X₁₂ Tyr Met where
- X₄ and X₁₁ are Tyr, His or Phe,
- X₃, X₈ and X₁₂ are Val, Met, Ile, Thr or Ala,
- X₆ is His, Gln, Arg, Lys, Ser or Gly,
- X₁ is His or Lys,
- X₉ is Asp, Glu or Lys ;
by a codon coding for a cysteine.

16. Vaccine according to claim 14 or 15, wherein the empty capsids of the foot-and-mouth disease virus comprise VP0, VP1, and VP3 proteins.

17. Vaccine according to anyone of claims 14-16, wherein the cDNA encoding P1 also codes for all or part of 2A.

18. Vaccine according to claim 17, wherein the cDNA also codes for part of 2B.

19. Vaccine according to any one of claims 14-18, wherein the cDNA encoding 3C also codes for all or part of proteins 3B, for a non-functional part of 3D, or for both all or part of proteins 3B and a non-functional part of 3D.

20. Vaccine according to claim 19, wherein the expression vector is a viral vector selected from the group consisting of poxviruses and replicatory adenoviruses.

21. Vaccine according to claim 20 wherein the expression vector is a poxvirus and the cDNA is under the control of a late promoter of viral origin selected from the group consisting of promoter P11K of the vaccine virus, promoter P28K of the vaccine virus, and promoter P160K ATI of the cowpox virus.

22. Vaccine according to any one of claims 14-19, wherein the expression vector is a plasmid vectorand the cDNA is under the control of a promoter selected from the group consisting of the early promoter of the SV40 virus, the promoter of the Rous sarcoma virus, and a promoter of a cytoskeleton gene.

23. Vaccine according to any one of claims 14-22, further comprising an adjuvant.

24. Vaccine according to claim 23, wherein said vaccine comprises a plasmid vector and the adjuvant comprises a cationic lipid containing a quaternary ammonium salt of formula:
R₁OCH₂CH(OR₁)CH₂N⁺(CH₃)₂(R₂)X
wherein R₁ is a linear, saturated or unsaturated aliphatic radical having 12 to 18 carbon atoms, R₂ is another aliphatic radical containing 2 or 3 carbon atoms, and X is an hydroxyl or amino group.

25. Vaccine according to claim 24, wherein the adjuvant comprises DMRIE.

26. Vaccine according to claim 25, wherein the DMRIE is associated with DOPE to form DMRIE-DOPE.

27. Vaccine according to claim 23, wherein said vaccine comprises a viral vector and the adjuvant comprises a polymer of acrylic or methacrylic acid, or a polymer of maleic anhydride and an alkenyl derivative.

28. Vaccine according to claim 23, wherein the adjuvant comprises GM-CSF.

29. Vaccine according to claim 23, wherein the adjuvant comprises a vector which expresses GM-CSF *in vivo*.

30. Vaccine according to any one of claims 1-29, wherein said vaccine is to be administered to cattle.

31. Vaccine according to any one of claims 1-30, for use in a method of vaccination of cattle.

32. Use of empty capsids of the foot-and-mouth disease virus obtained by expression *in vitro,* in eukaryotic cells, of the cDNA encoding the P1 region of the foot-and-mouth virus genome and the cDNA of the region of the foot-and-mouth virus genome encoding the 3C protease, for the manufacture of a vaccine against foot-and-mouth disease for administration to cattle, wherein the cDNA of the P1 region of the foot-and-mouth disease virus genome is modified by replacement of the codon coding for amino acid 179 of the amino acid sequence as set forth in SEQ ID NO:38 or of the codon coding for the amino acid corresponding to amino acid 179 of the amino acid sequence as set forth in SEQ ID NO:38 by a codon coding for a cysteine.

## Patentansprüche

1. Impfstoff gegen die Maul- und Klauenseuche, umfassend eine wirksame Menge an Antigen, das aus leeren Capsiden des Maul-und-Klauenseuche-Virus besteht, die in vitro durch Expression der cDNA für die Region P1 des Genoms des Maul-und-Klauenseuche-Virus, die für das Capsid kodiert, und der cDNA für die Region des Genoms des Maul-und-Klauenseuche-Virus, die für die Protease 3C kodiert, in eukaryotischen Zellen erhalten wird, wobei der Impfstoff außerdem ein Vehikel oder einen Excipienten umfasst, das/der auf veterinärmedizinischer Ebene pharmazeutisch annehmbar ist, wobei die cDNA für die Region P1 des Genoms des Maul-und-Klauenseuche-Virus modifiziert ist, indem das Codon, das für die Aminosäure 179 der Aminosäuresequenz SEQ ID NR: 38 kodiert, oder das Codon, das für die Aminosäure kodiert, die der Aminosäure 179 der Aminosäuresequenz SEQ ID NR: 38 entspricht, durch ein für Cystein kodierendes Codon ersetzt worden ist.

2. Impfstoff nach Anspruch 1, wobei die cDNA für die Region P1 des Genoms des Maul-und-Klauenseuche-Virus modifiziert ist, indem das Codon, das für die Aminosäure X6 der Sequenz X1 Gly X3 X4 Gly X6 Leu X8 X9 Ser X11 X12 Tyr Met, worin
- X4 und X11 für Tyr, His oder Phe stehen,
- X3, X8 und X12 für Val, Met, Ile, Thr oder Ala
stehen,
- X6 für His, Gln, Arg, Lys, Ser oder Gly steht,
- X1 für His oder Lys steht,
- X9 für Asp, Glu oder Lys steht,
kodiert, durch ein für Cystein kodierendes Codon ersetzt ist.

3. Impfstoff nach Anspruch 1 oder 2, wobei die leeren Capside des Maul-und-Klauenseuche-Virus die Proteine VP0, VP1 und VP3 umfassen.

4. Impfstoff nach einem der Ansprüche 1 bis 3, wobei die für P1 kodierende cDNA auch für das gesamte oder einen Teil von 2A kodiert.

5. Impfstoff nach Anspruch 4, wobei die cDNA auch für einen Teil von 2B kodiert.

6. Impfstoff nach einem der Ansprüche 1 bis 5, wobei die für 3C kodierende cDNA auch für das gesamte oder einen Teil der Proteine 3B, für einen nicht-funktionellen Teil von 3D oder gleichzeitig für das gesamte oder einen Teil der Proteine 3B und für einen nicht-funktionellen Teil von 3D kodiert.

7. Impfstoff nach einem der Ansprüche 1 bis 6, wobei die leeren Capside in Form von Untereinheiten vorliegen, die durch Expression der cDNA in eukaryotischen Zellen durch einen in vitro-Expressionsvektor in Abhängigkeit von einem induzierbaren Promotor oder aus einem Virus stammenden späten Promotor erhalten werden.

8. Impfstoff nach Anspruch 7, wobei es sich bei dem Promotor um den Promotor des Bakteriophagen T7 handelt und die leeren Capside in Form von Untereinheiten durch Expression der cDNA in Gegenwart der Expression von T7-Polymerase erhalten werden.

9. Impfstoff nach einem der vorhergehenden Ansprüche, wobei der Expressionsvektor das Vacciniavirus ist.

10. Impfstoff nach einem der vorhergehenden Ansprüche, wobei der Impfstoff in einem Medium des DMEM-Typs formuliert ist, das die Aufbewahrung der leeren Capside ermöglicht.

11. Impfstoff nach einem der Ansprüche 1 bis 10, der außerdem ein Adjuvans umfasst.

12. Impfstoff nach Anspruch 11, wobei der Impfstoff als Adjuvans Aluminiumhydroxid, Saponin, Avridin, DDA, ein Acryl- oder Methacrylsäurepolymer, ein Polymer aus Maleinsäureanhydrid und Alkenylderivat oder GM-CSF umfasst oder wobei der Impfstoff in Form einer Wasser-in-Öl-, Öl-in-Wasser- oder Wasser-in-Öl-in-Wasser-Emulsion formuliert ist.

13. Impfstoff nach Anspruch 12, wobei das Adjuvans ein Carbomer umfasst.

14. Impfstoff gegen die Maul- und Klauenseuche, umfassend einen Expressionsvektor, der die cDNA für die Region P1 des Genoms des Maul-und-Klauenseuche-Virus, die für das Capsid kodiert, und die cDNA für die Region des Genoms des Maul-und-Klauenseuche-Virus, die für die Protease 3C kodiert, enthält, so dass in vivo eine wirksame Menge an Antigen hergestellt wird, das aus leeren Capsiden des Maul-und-Klauenseuche-Virus besteht, wobei der Impfstoff außerdem ein Vehikel oder einen Excipienten umfasst, das/der auf veterinärmedizinischer Ebene pharmazeutisch annehmbar ist, wobei die cDNA für die Region P1 des Genoms des Maul-und-Klauenseuche-Virus modifiziert ist, indem das Codon, das für die Aminosäure 179 der Aminosäuresequenz SEQ ID NR: 38 kodiert, oder das Codon, das für die Aminosäure kodiert, die der Aminosäure 179 der Aminosäuresequenz SEQ ID NR: 38 entspricht, durch ein für Cystein kodierendes Codon ersetzt worden ist.

15. Impfstoff nach Anspruch 14, wobei die cDNA für die Region P1 des Genoms des Maul-und-Klauenseuche-Virus modifiziert ist, indem das Codon, das für die Aminosäure X6 der Sequenz X1 Gly X3 X4 Gly X6 Leu X8 X9 Ser X11 X12 Tyr Met, worin
- X4 und X11 für Tyr, His oder Phe stehen,
- X3, X8 und X12 für Val, Met, Ile, Thr oder Ala
stehen,
- X6 für His, Gln, Arg, Lys, Ser oder Gly steht,
- X1 für His oder Lys steht,
- X9 für Asp, Glu oder Lys steht,
kodiert, durch ein für Cystein kodierendes Codon ersetzt ist.

16. Impfstoff nach Anspruch 14 oder 15, wobei die leeren Capside des Maul-und-Klauenseuche-Virus die Proteine VP0, VP1 und VP3 umfassen.

17. Impfstoff nach einem der Ansprüche 14 bis 16, wobei die für P1 kodierende cDNA auch für das gesamte oder einen Teil von 2A kodiert.

18. Impfstoff nach Anspruch 17, wobei die cDNA auch für einen Teil von 2B kodiert.

19. Impfstoff nach einem der Ansprüche 14 bis 18, wobei die für 3C kodierende cDNA auch für das gesamte oder einen Teil der Proteine 3B, für einen nicht-funktionellen Teil von 3D oder gleichzeitig für das gesamte oder einen Teil der Proteine 3B und für einen nicht-funktionellen Teil von 3D kodiert.

20. Impfstoff nach Anspruch 19, wobei der Expressionsvektor ein viraler Vektor ist, der aus replizierenden Pockenviren oder Adenoviren ausgewählt wird.

21. Impfstoff nach Anspruch 20, wobei der Expressionsvektor ein Pockenvirus ist und die cDNA unter der Abhängigkeit eines aus Virus stammenden späten Promotors steht, der aus P11K des Vacciniavirus, P28K des Vacciniavirus und P160K des Kuhpockenvirus ausgewählt wird.

22. Impfstoff nach einem der Ansprüche 14 bis 19, wobei der Expressionsvektor ein Plasmidvektor ist und die cDNA unter der Abhängigkeit eines Promotors steht, der aus der Gruppe ausgewählt wird, die aus dem frühen Promotor des SV40-Virus, dem LTR-Promotor des Rous-Sarkomvirus und einem Promotor eines Zytoskelettgens besteht.

23. Impfstoff nach einem der Ansprüche 14 bis 22, der außerdem ein Adjuvans enthält.

24. Impfstoff nach Anspruch 23, wobei der Impfstoff einen Plasmidvektor umfasst und das Adjuvans ein kationisches Lipid umfasst, das ein quaternäres Ammoniumsalz der Formel
R1OCH2CH(OR1)CH2N+(CH3)2(R2)X
umfasst, worin R1 ein gerader gesättigter oder ungesättigter aliphatischer Rest mit 12 bis 18 Kohlenstoffatomen ist, R2 ein anderer aliphatischer Rest ist, der 2 oder 3 Kohlenstoffatome enthält, und X eine Hydroxyl- oder Amingruppe ist.

25. Impfstoff nach Anspruch 24, wobei das Adjuvans DMRIE umfasst.

26. Impfstoff nach Anspruch 25, wobei das DMRIE mit DOPE unter Bildung von DMRIE-DOPE vereinigt ist.

27. Impfstoff nach Anspruch 23, wobei der Impfstoff einen viralen Vektor umfasst und das Adjuvans ein Acrylsäure- oder Methacrylsäure-Polymer oder ein Polymer aus Maleinsäureanhydrid und Alkenylderivat umfasst.

28. Impfstoff nach Anspruch 23, wobei das Adjuvans GM-CSF umfasst.

29. Impfstoff nach Anspruch 23, wobei das Adjuvans einen Vektor umfasst, der GM-CSF in vivo exprimiert.

30. Impfstoff nach einem der Ansprüche 1 bis 29, wobei der Impfstoff für die Verabreichung an ein Rind bestimmt ist.

31. Impfstoff nach einem der Ansprüche 1 bis 30 für die Verwendung bei einem Verfahren zur Impfung eines Rinds.

32. Verwendung leerer Capside des Maul-und-Klauenseuche-Virus, die in vitro durch Expression der cDNA für die Region P1 des Genoms des Maul-und-Klauenseuche-Virus, die für das Capsid kodiert, und der cDNA für die Region des Genoms des Maul-und-Klauenseuche-Virus, die für die Protease 3C kodiert, in eukaryotischen Zellen erhalten wird, zur Herstellung eines Impfstoffs gegen die Maul- und Klauen-Seuche, der für die Verabreichung an ein Rind bestimmt ist, wobei die cDNA für die Region P1 des Genoms des Maul-und-Klauenseuche-Virus modifiziert ist, indem das Codon, das für die Aminosäure 179 der Aminosäuresequenz SEQ ID NR: 38 kodiert, oder das Codon, das für die Aminosäure kodiert, die der Aminosäure 179 der Aminosäuresequenz SEQ ID NR: 38 entspricht, durch ein für Cystein kodierendes Codon ersetzt worden ist.
